# EUROPEAN PATENT APPLICATION

(11) **EP 1 698 621 A1**
(43) Date of publication of application: **06.09.2006**
(21) Application number: 04807936.2
(22) Date of filing: 27.12.2004
(51) Int. Cl.: C07D 207/12

(54) **METHOD FOR PRODUCING PYRROLIDINE DERIVATIVE**

(30) Priority: 26.12.2003 JP 2003431686
(71) Applicant: DAIICHI PHARMACEUTICAL CO., LTD., Chuo-ku, Tokyo 103-8234 (JP)
(72) Inventor: TAKAYANAGI, Y., Daiichi Pharmaceutical Co.,Ltd., Edogawa-ku, Tokyo 1348630 (JP); YAMADA,T, Daiichi Pure Chemicals Co.,Ltd., Iwate-gun, Iwate 0287305 (JP); FURUYA, Yukito, Daiichi Pharmaceutical Co., Ltd, Edogawa-ku, Tokyo 1348630 (JP); YONEDA, Yoshiyuki, Daiichi Pharmaceutical Co.,Ltd, Edogawa-ku, Tokyo 1348630 (JP)
(74) Representative: Hartz, Nikolai
(86) International application number: PCT/JP2004/019581
(87) International publication number: WO 2005/066124

(57) **Abstract**

The present invention provides an advantageous method for producing an intermediate which is useful for production of a compound which exhibits excellent VLA-4 inhibitory effect and safety.

An intermediate (14) is produced through the following reaction scheme.

## Description

### Technical Field

The present invention relates to a method for producing a compound which is useful as an intermediate for production of a compound which exhibits excellent VLA-4 inhibitory effect and safety.

### Background Art

VLA-4 is a molecule that is involved in cell adhesion and expressed on monocytes, lymphocytes, eosinophils and basophils. VLA-4 is known to act as a receptor of Vascular cell adhesion molecule-1 (VCAM-1) or the like.

In recent years, it has been reported that, the selective inhibition of cell adhesion mediated by VLA-4 and VCAM-1 might become a resolution method for the treatment of autoimmune diseases or allergic inflammatory diseases.

A compound represented by formula (I) described in Patent Document 1; e.g., a compound represented by the following formula (1), or a salt thereof is expected to be used as a dug compound because of its anti-inflammatory effect based on excellent VLA-4 inhibitory effect, as well as its high safety (see Patent Document 1).

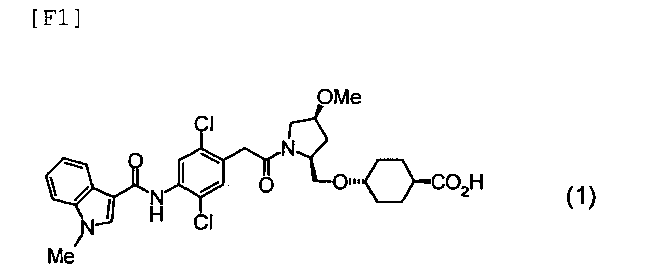

A compound represented by the following formula (2):

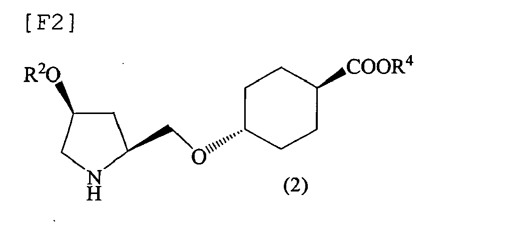

(wherein R² represents a lower alkyl group, and R⁴ represents an alkyl group which may be substituted, or an aralkyl group which may be substituted) is an important intermediate for the production of the compound represented by formula (I) disclosed in Patent Document 1.
In a conventional method for producing the compound, the carboxylic acid moiety of trans-4-hydroxy-L-proline whose nitrogen atom has been protected is converted to a hydroxymethyl group and then to a sulfonyl group; a benzoic acid unit is introduced to the product; the secondary hydroxy group is inverted through the Mitsunobu reaction to thereby form a methyl ether; the protecting group on the nitrogen atom is removed; the benzene ring of the benzoic acid moiety is reduced; and the product is isomerized through use of a metal base. Thus, the compound represented by formula (2) is obtained as an oily product or as crystals in the form of a trifluoroacetic acid salt or a hydrochloric acid salt (see Patent Documents 1 and 2).

However, there remain some problems in that the above conventional method is not suitable for mass production. For example, this method is required to employ a Mitsunobu reaction for which the use of an explosive azo reagent is necessary and a purification step by column chromatography.

Furthermore, even when a mixture obtained through reduction of a benzene ring and predominantly containing a cis-form compound is subjected to isomerization, the ratio of the cis-form compound to the trans-form compound obtained is limited to around 1:1. Thus the conventional method is still problematic, because the obtainable maximum amount of the target trans-form compound is half amount of the product after separation by column chromatography.
[Patent Document 1] WO2002/053534
[Patent Document 2] WO 2004/099136

### Disclosure of the Invention

### Problems to be Solved by the Invention

The present invention is directed to provide an industrially advantageous method for producing a compound represented by the above formula (2), the compound being an important intermediate for the production of a compound which exhibits excellent VLA-4 inhibitory effect and safety.

### Means for Solving the Problems

In view of the foregoing, the present inventors have performed extensive studies, and have found that the compound represented by the above formula (2), which is an important intermediate, can be produced as crystals in the form of an acid-salt compound by use of commercially available cis-4-hydroxy-L-proline as a starting material through the following process: in the presence of a base, while racemization is prevented, the starting material is alkylated in one pot; the product is reduced and then reacted with an arylsulfonyl chloride or an alkylsulfonyl chloride in the presence of a base to thereby produce a compound represented by the following formula (3):

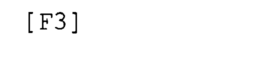

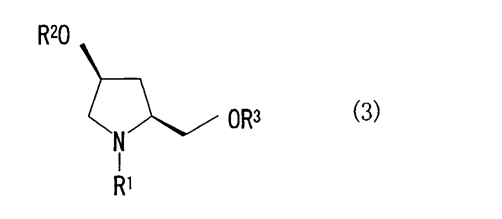

(wherein R¹ represents a protecting group for the amino group, R² has the same meaning as defined above, and R³ represents an arylsulfonyl group which may be substituted or an alkylsulfonyl group which may be substituted); the product is subjected to Sn2 reaction with a 4-hydroxybenzoic acid ester to thereby produce a compound represented by the following formula (4):

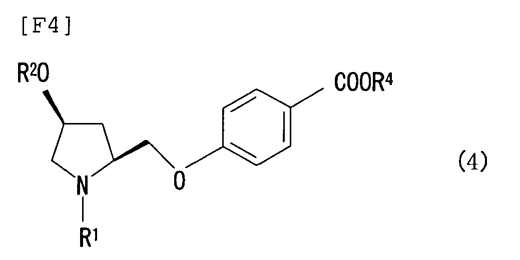

(wherein R¹, R², and R⁴ have the same meanings as defined above); and R¹ is removed. The above process can be performed with no Mitsunobu reaction reagent. In addition, since the compound is obtained as crystals, purification through column chromatography can be eliminated.

The product obtained through reduction of the thus-produced compound represented by the above formula (4) predominantly contains a cis-form compound. In order to obtain the target trans-form compound, isomerization must be performed to convert the cis-form compound to the corresponding trans-form compound. In terms of isomerization, the present inventors also found that the ratio of the trans-form to the cis-form can be increased to about 4:1 through isomerization by use of a base and then addition of water for hydrolysis to the reaction system. The present inventors also found that, through addition of camphorsulfonic acid, the compound represented by the above formula (2) can preferentially be obtained as crystals. Thus, purification through column chromatography can be eliminated, and only trans-form compound can be obtained with precedence.

These excellent improvements provide more efficient production of an important intermediate represented by the above formula (2). The present invention has been accomplished on the basis of these findings.

The present invention provides a method for producing a compound represented by formula (f): 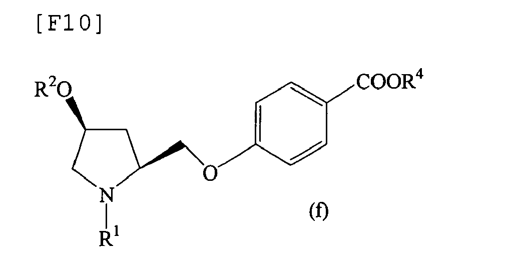 (wherein R¹ represents a protecting group for the amino group, R² represents a lower alkyl group, and R⁴ represents an alkyl group which may be substituted or an aralkyl group which may be substiuted), characterized by comprising reacting an alkyl halide with a compound represented by formula (a): 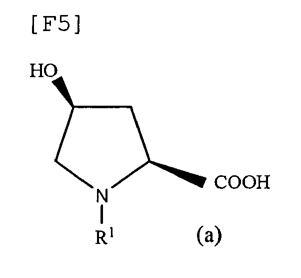 (wherein R¹ has the same meaning as defined above) in the presence of a base to thereby produce a compound represented by formula (b): 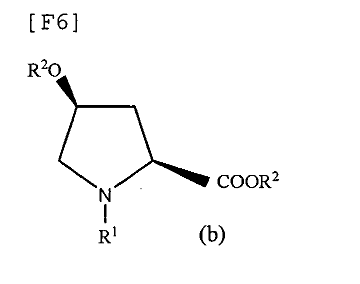 (wherein R¹ and R² have the same meanings as defined above); reacting a reducing agent with the compound represented by formula (b) to thereby produce a compound represented by formula (c): 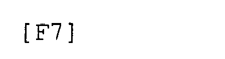 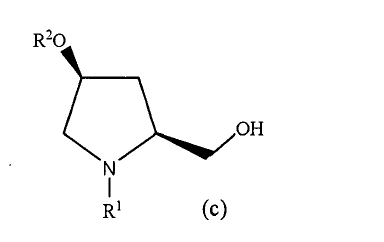 (wherein R¹ and R² have the same meanings as defined above); reacting the compound represented by formula (c) with an arylsulfonyl halide which may be substituted or an alkylsulfonyl halide which may be substituted in the presence of a base to thereby produce a compound represented by formula (d): 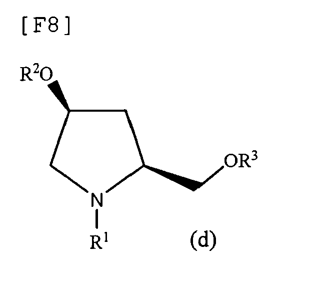 (wherein R¹ and R² have the same meanings as defined above, and R³ represents an arylsulfonyl group which may be substituted or an alkylsulfonyl group which may be substituted); and reacting the compound represented by formula (d) with a compound represented by formula (e): 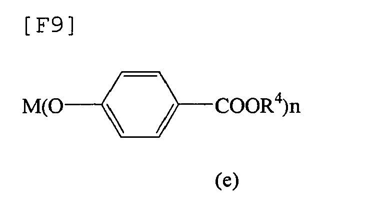 (wherein R⁴ has the same meaning as defined above, M represents an alkali metal atom or an alkaline earth metal atom, and n denotes an integer of 1 or 2).

The present invention also provides an oxalic acid salt of the compound represented by formula (g).

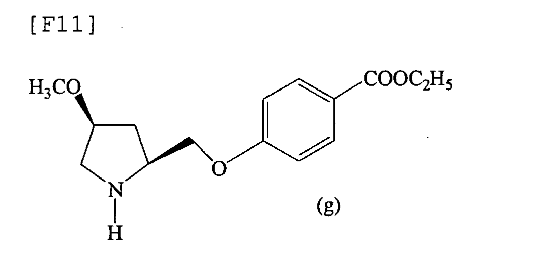

The present invention also provides a method for producing a compound represented by formula (l): 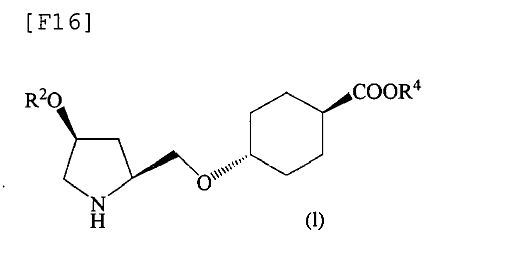 (wherein R² and R⁴ have the same meanings as defined above), characterized by comprising reducing a compound represented by formula (h): 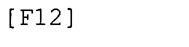 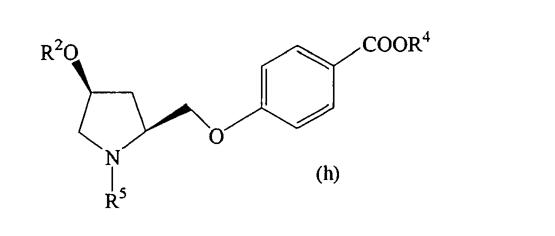 (wherein R² and R⁴ have the same meanings as defined above, and R⁵ represents a hydrogen atom or a protecting group for the amino group) to thereby produce a compound represented by formula (i): 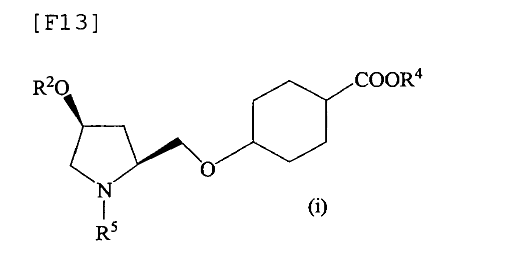 (wherein R², R⁴, and R⁵ have the same meanings as defined above); treating the compound represented by formula (i) with a base in an aprotic polar solvent and then reacting with water to thereby produce a compound represented by formula (j): 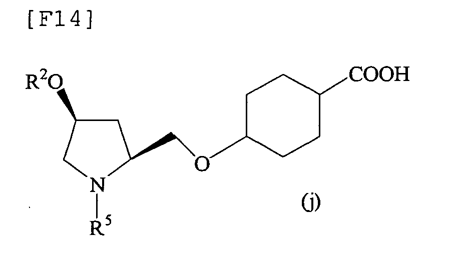 (wherein R² and R⁵ have the same meanings as defined above); and treating the compound represented by formula (j) with an acid in the presence of an alcohol to thereby produce a compound represented by formula (k): 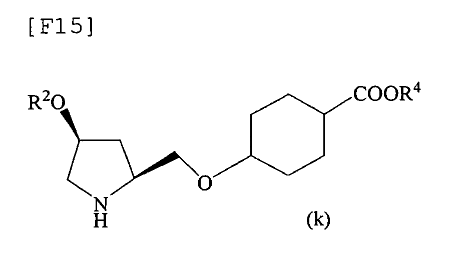 (wherein R² and R⁴ have the same meanings as defined above); and treating the compound represented by formula (k) with camphorsulfonic acid to thereby form an acid adduct salt so that an isomer of interest can be isolated.

The present invention also provides a method for producing a compound represented by formula (l): 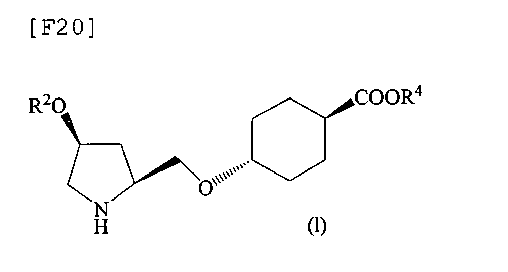 (wherein R² and R⁴ have the same meanings as defined above), characterized by comprising reducing a compound represented by formula (h): 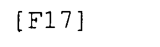 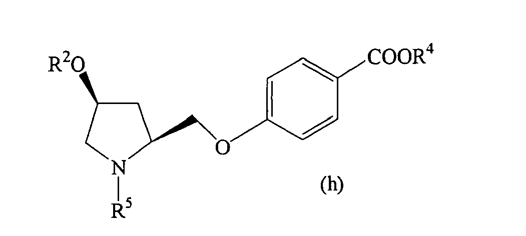 (wherein R², R⁴, and R⁵ have the same meanings as defined above) to thereby produce a compound represented by formula (i): 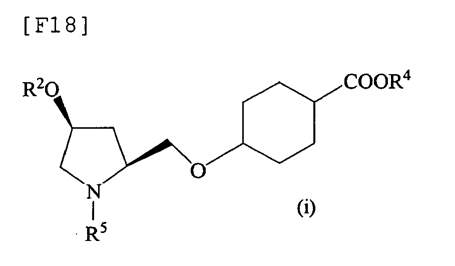 (wherein R², R⁴, and R⁵ have the same meanings as defined above); treating the compound represented by formula (i) with a base in an aprotic polar solvent and then reacting with water to thereby produce a compound represented by formula (j): 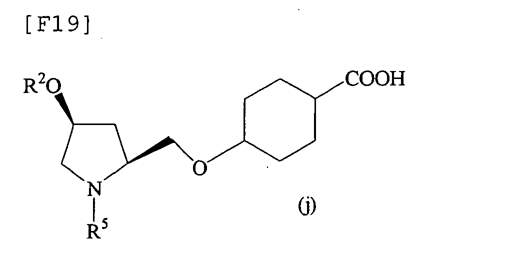 (wherein R² and R⁵ have the same meanings as defined above); and treating the compound represented by formula (j) with camphorsulfonic acid to thereby form an acid adduct salt so that an isomer of interest can be isolated.

The present invention also provides a method for producing a compound represented by formula (l): 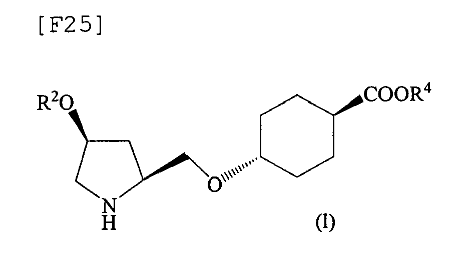 (wherein R² and R⁴ have the same meanings as defined above), characterized by comprising reducing a compound represented by formula (h): 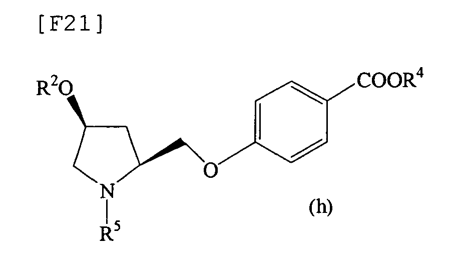 (wherein R², R⁴, and R⁵ have the same meanings as defined above) to thereby produce a compound represented by formula (i): 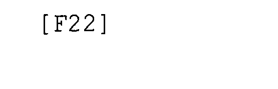 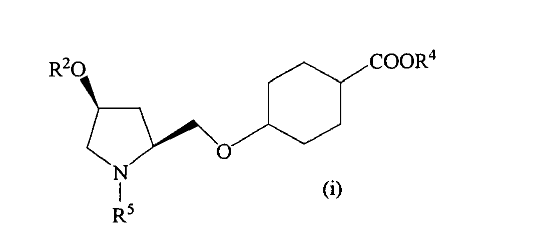 (wherein R², R⁴, and R⁵ have the same meanings as defined above); treating the compound represented by formula (i) with a base in an aprotic polar solvent and then reacting with water to thereby produce a compound represented by formula (j) : 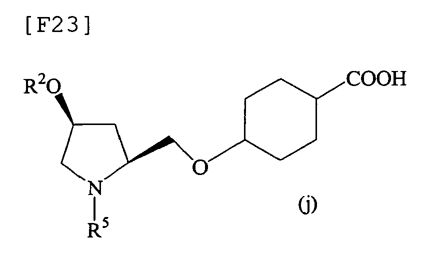 (wherein R² and R⁵ have the same meanings as defined above); isolating the compound represented by formula (j) as a salt and then treating the salt with an acid in the presence of an alcohol to thereby produce a compound represented by formula (k): 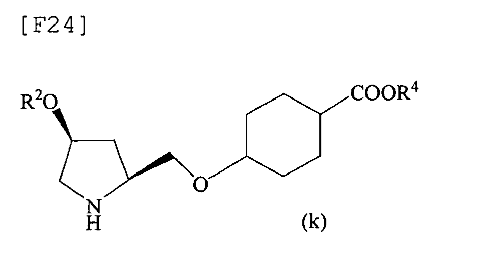 (wherein R² and R⁴ have the same meanings as defined above); and treating the compound represented by formula (k) with camphorsulfonic acid to thereby form an acid adduct salt so that an isomer of interest can be isolated.

The present invention also provides a method for producing a compound represented by formula (l): 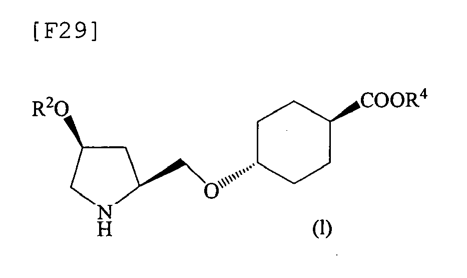 (wherein R² and R⁴ have the same meanings as defined above), characterized by comprising reducing a compound represented by formula (h): 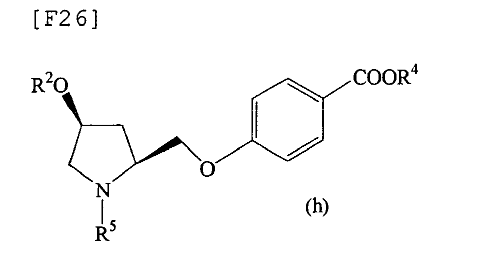 (wherein R², R⁴, and R⁵ have the same meanings as defined above) to thereby produce a compound represented by formula (i) : 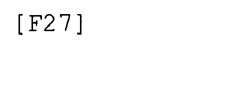 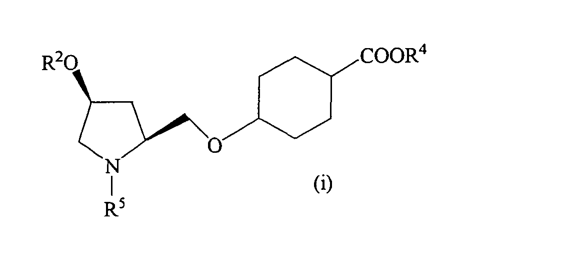 (wherein R², R⁴, and R⁵ have the same meanings as defined above); treating the compound represented by formula (i) with a base in an aprotic polar solvent and then reacting with water to thereby produce a compound represented by formula (j): 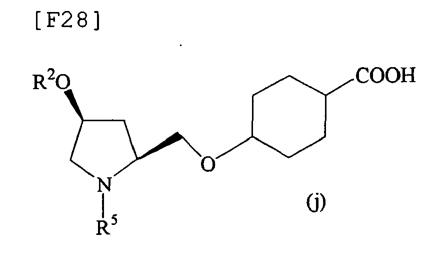 (wherein R² and R⁵ have the same meanings as defined above); isolating the compound represented by formula (j) as a salt and then treating the compound with camphorsulfonic acid to thereby form an acid adduct salt so that an isomer of interest can be isolated.

The present invention also provides a method for producing a compound represented by formula (l) from a compound represented by formula (h) serving as a starting material through any of the above methods, wherein the compound represented by formula (h) is a compound represented by formula (f) produced through the above method, a compound produced through removal of the protecting group for the amino group of a compound represented by formula (f), or a compound produced through removal of the protecting group for the amino group of a compound represented by formula (f) and then protection of the amino group with a protecting group which differs from the removed protecting group.

The present invention also provides a camphorsulfonic acid salt of the compound represented by formula (m).

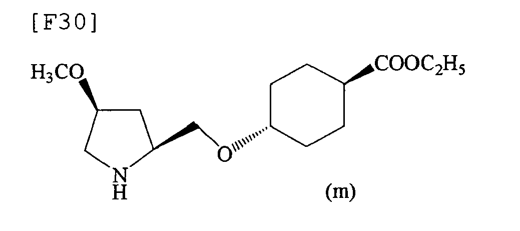

The present invention also provides a method for producing a compound represented by formula (o): 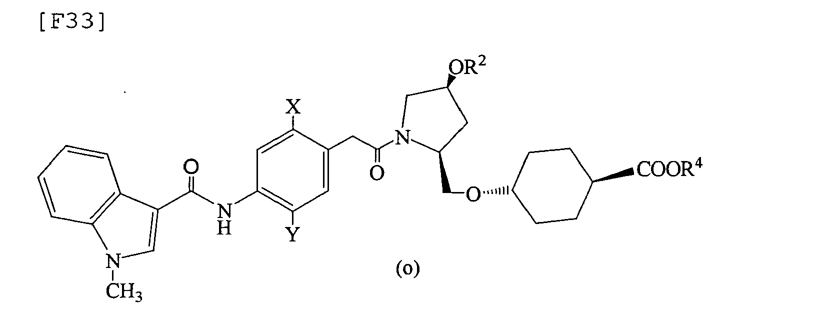 (wherein R² and R⁴ have the same meanings as defined above, X represents a hydrogen atom or a halogen atom, and Y represents a halogen atom or a lower alkoxy group), characterized by comprising reacting a compound represented by formula (l): 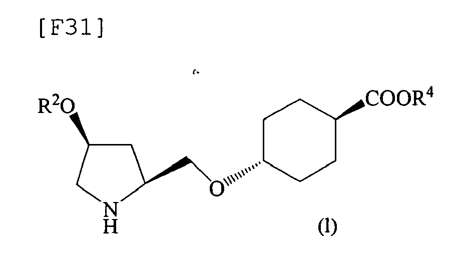 (wherein R² and R⁴ have the same meanings as defined above) produced through any of the above methods, with a compound represented by formula (n): 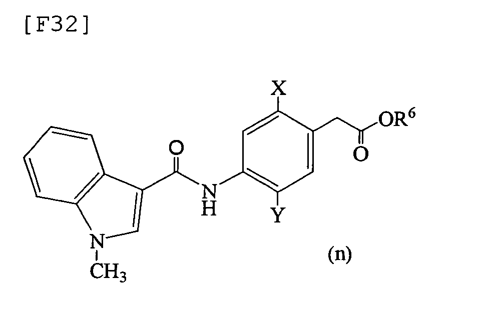 (wherein R⁶ represents a hydrogen atom, a linear or branched lower alkyl group which may be substituted, or an aralkyl group which may be substituted, and X and Y have the same meanings as defined above).

The present invention also provides a method for producing a compound represented by formula (p): 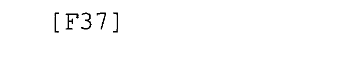 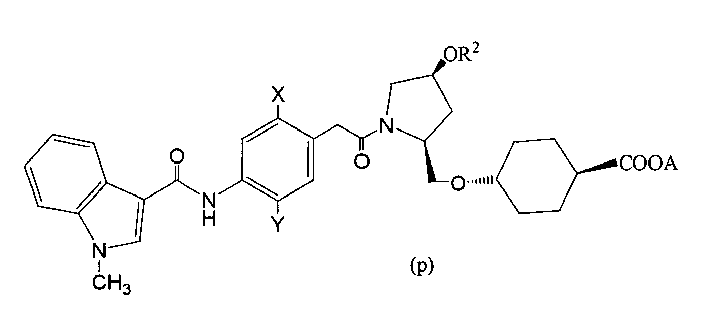 (wherein A represents a hydrogen atom, an alkali metal atom, an alkaline earth metal atom, or an organic amine, and R², X, and Y have the same meanings as defined above) or a hydrate thereof, characterized by comprising reacting a compound represented by formula (l): 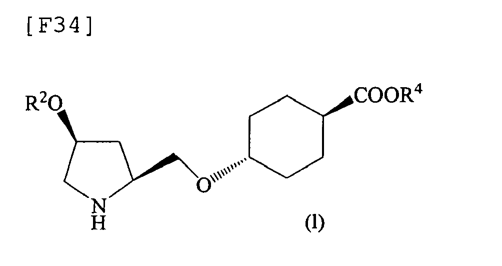 (wherein R² and R⁴ have the same meanings as defined above) produced through any of the above methods, with a compound represented by formula (n): 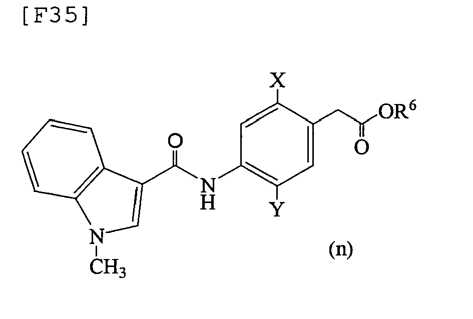 (wherein R⁶, X, and Y have the same meanings as defined above) to thereby produce a compound represented by formula (o): 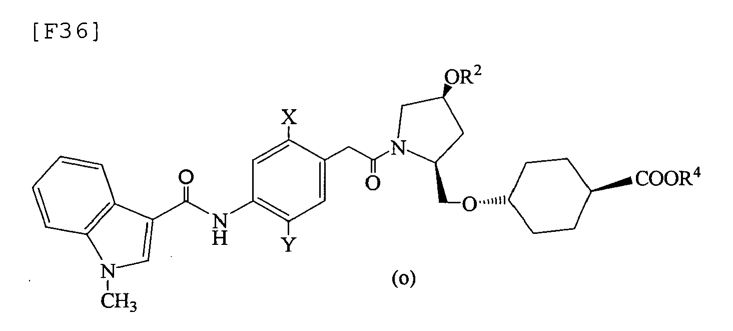 (wherein R², R⁴, X, and Y have the same meanings as defined above); and hydrolyzing the compound represented by formula (o).

The present invention also provides a method for producing a compound represented by formula (o): 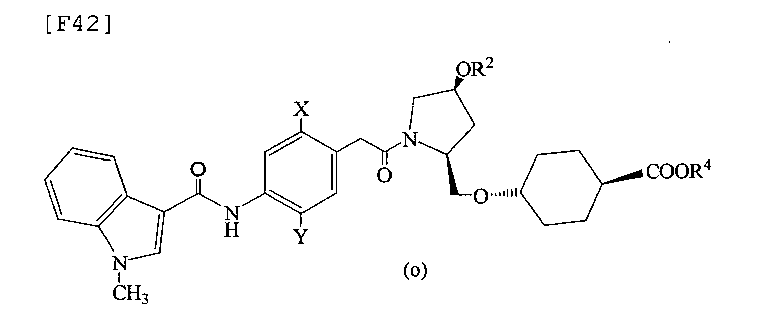 (wherein R², R⁴, X, and Y have the same meanings as defined above), characterized by comprising reacting a compound represented by formula (l): 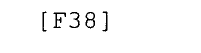 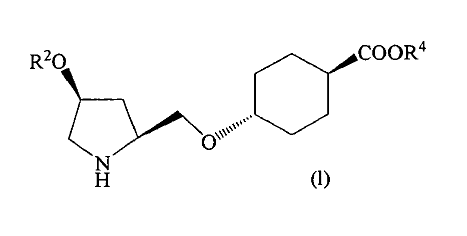 (wherein R² and R⁴ have the same meanings as defined above) produced through any of the above methods, with a compound represented by formula (s): 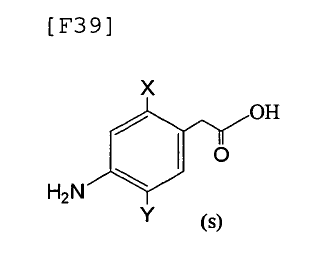 (wherein X and Y have the same meanings as defined above) to thereby produce a compound represented by formula (t): 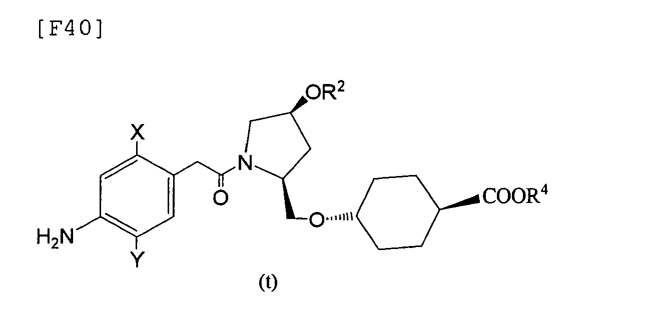 (wherein R², R⁴, X, and Y have the same meanings as defined above); and reacting the compound represented by formula (t) with a compound represented by formula (u).

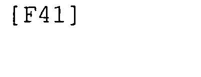

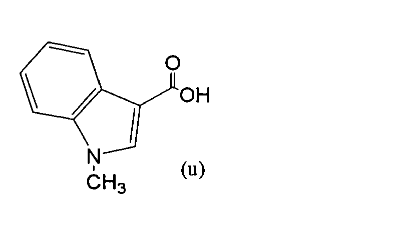

The present invention also provides a method for producing a compound represented by formula (p): 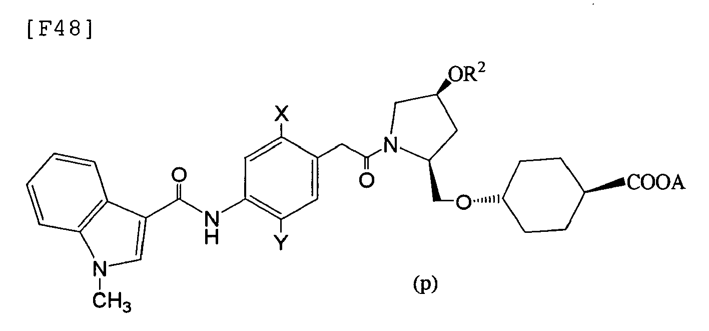 (wherein R², X, Y, and A have the same meanings as defined above) or a hydrate thereof, characterized by comprising reacting a compound represented by formula (l) : 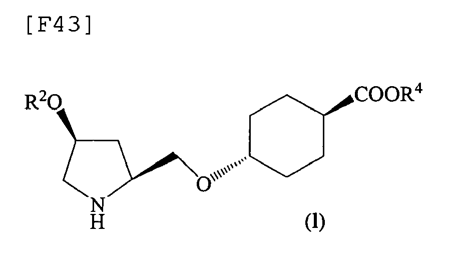 (wherein R² and R⁴ have the same meanings as defined above) produced through any of the above methods, with a compound represented by formula (s): 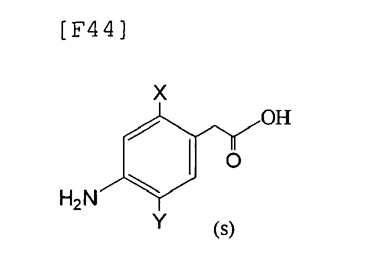 (wherein X and Y have the same meanings as defined above) to thereby produce a compound represented by formula (t): 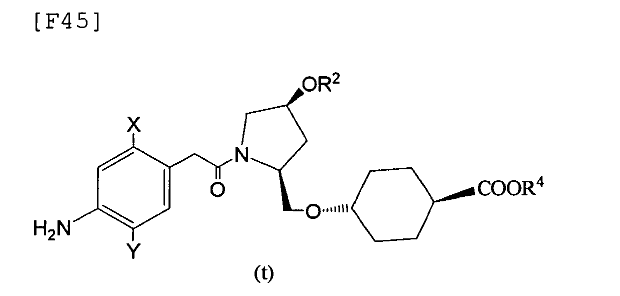 (wherein R², R⁴, X, and Y have the same meanings as defined above); reacting the compound represented by formula (t) with a compound represented by formula (u): 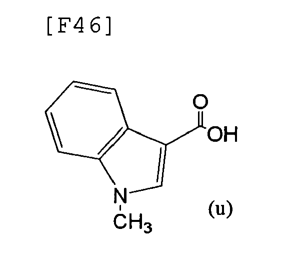 to thereby produce a compound represented by formula (o): 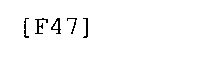 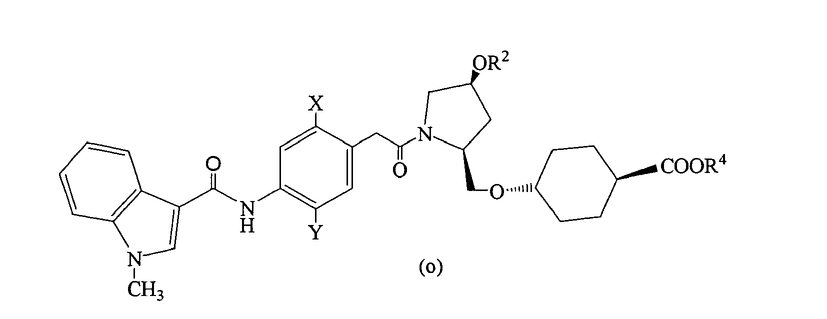 (wherein R², R⁴, X, and Y have the same meanings as defined above); and hydrolyzing the compound represented by formula (o).

### Effects of the Invention

The methods of the present invention enables efficient production of a drug compound represented by formula (I) described in Patent Document 1, the compound exhibiting anti-inflammatory effect based on its excellent VLA-4 inhibitory effect and being highly safety.

### Best Modes for Carrying Out the Invention

The present invention will next be described in detail. In this specification, the term "lower alkyl group" refers to a C1-C6 alkyl group.

In one preferred embodiment, the present invention employs the following reaction scheme starting with cis-4-hydroxy-L-proline, which is commercially available:

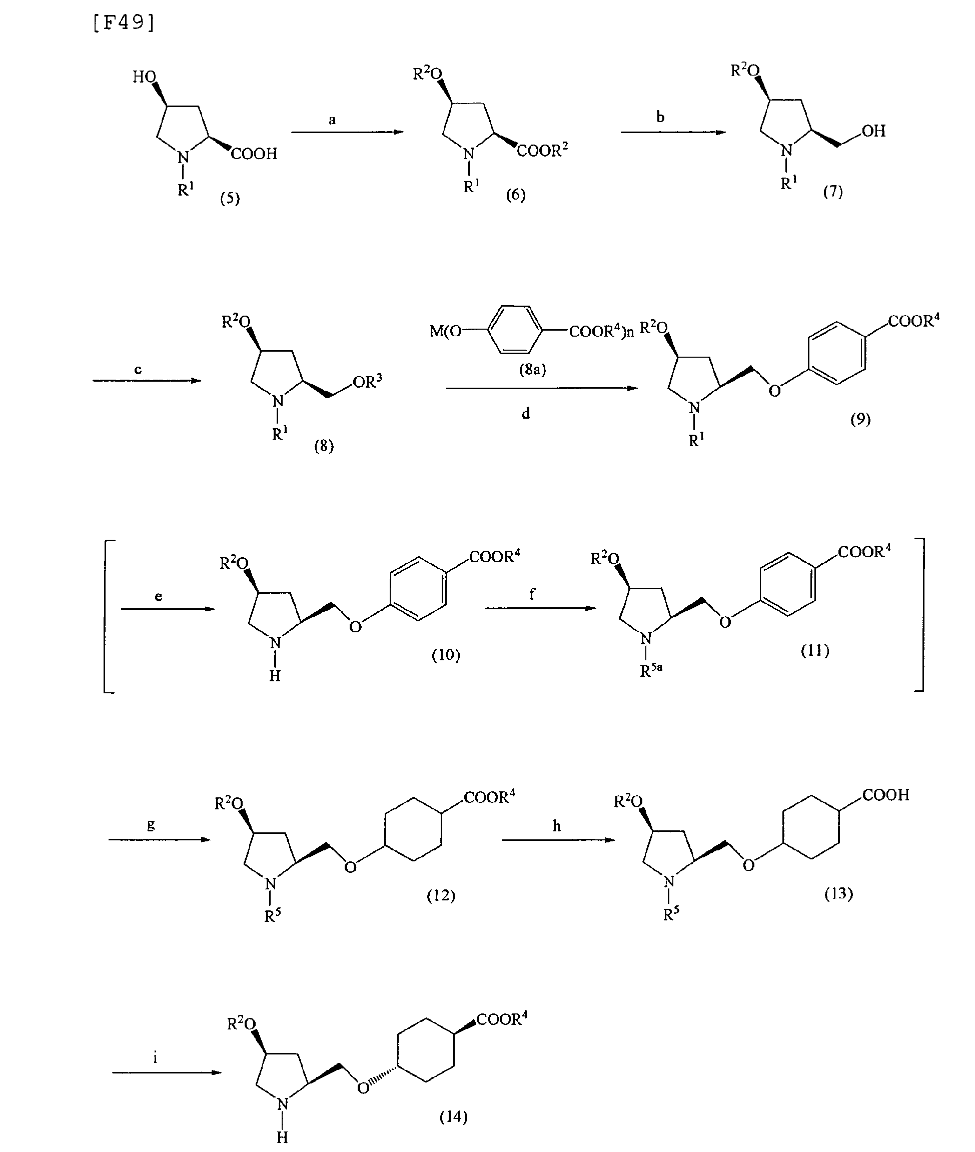

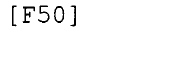

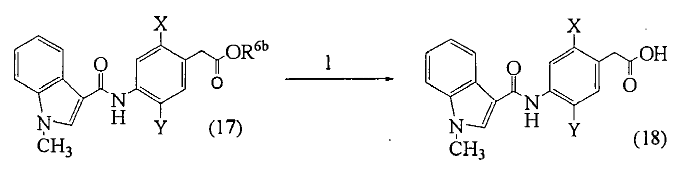

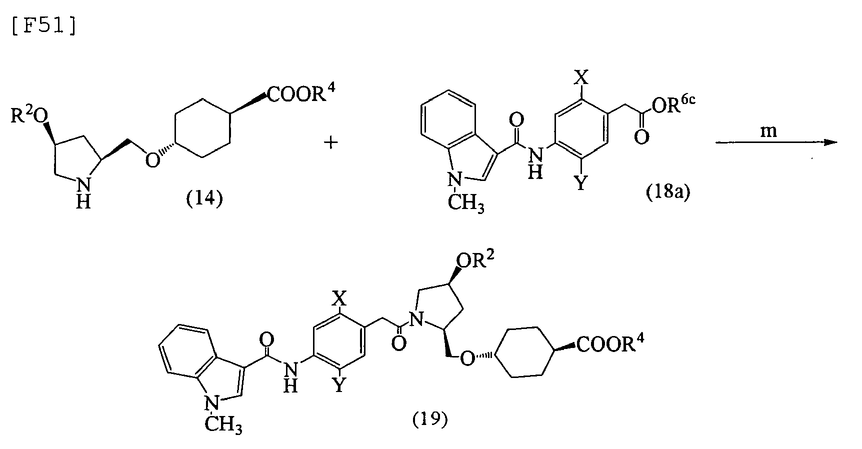

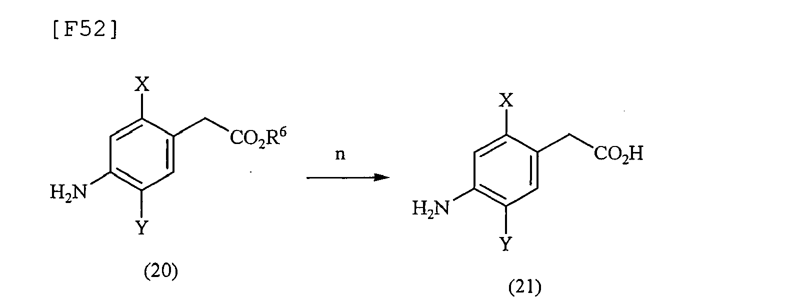

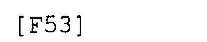

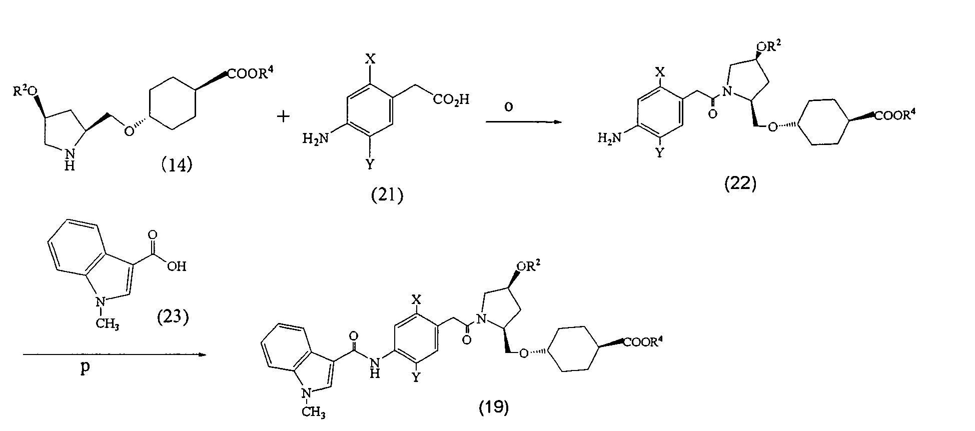

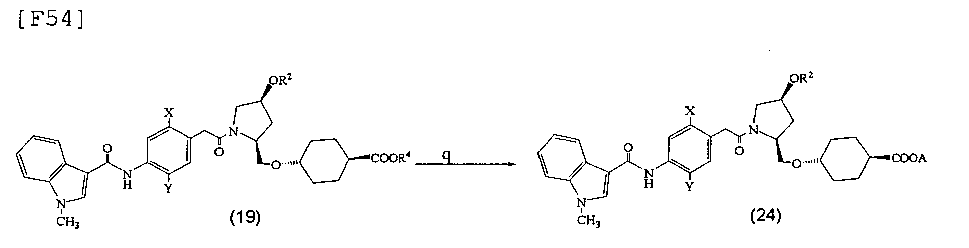

(wherein R^{5a} represents a protecting group for the amino group; R⁶ represents a hydrogen atom, a linear or branched lower alkyl group which may be substituted, or an aralkyl group which may be substituted; R^{6b} represents a linear or branched lower alkyl group which may be substituted or an aralkyl group which may be substituted; R^{6c} represents a hydrogen atom or R^{6b}; and R¹ to R⁵, X, Y, n, M, and A have the same meanings as defined above).
Each step of the above reaction scheme will next be described in detail.

### [Step a]

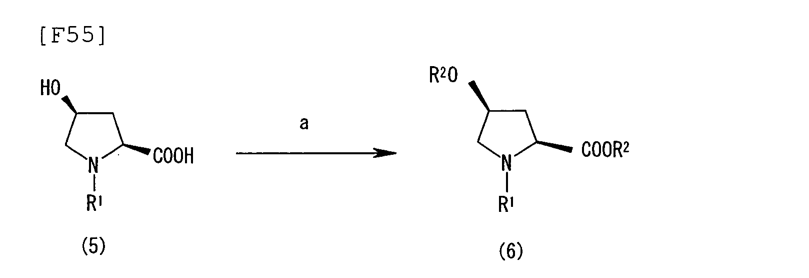

In step a, the hydroxy group of a compound represented by formula (5) is converted into an alkoxy group, and the carboxylic acid is converted into an ester. Specifically, a compound represented by formula (5) is reacted with an alkyl halide in the presence of a base, to thereby yield a compound represented by formula (6).

In formulas (5) and (6), R¹ represents a protecting group of the nitrogen atom (for the amino group). Preferably, the protecting group is, for example, a group described in "Protective Groups in Organic Synthesis, edited by T. W. Greene and P. G. Wuts, John Wiley&Sons, Inc., New York, 1991." Specific examples of the protecting group include carbonate groups, acyl groups, alkyl groups, and aralkyl groups. Of these, carbonate groups such as alkyloxycarbonyl groups (e.g., substituted or non-substituted aralkyloxycarbonyl groups, tert-butoxycarbonyl group) are preferred. Among them, aralkyloxycarbonyl groups are preferred, with benzyloxycarbonyl being particularly preferred. R² represents a lower alkyl group, with methyl and ethyl being particularly preferred.

In step a, the compound represented by formula (6) may be produced through reaction of a compound represented by formula (5) with a base in a solvent and addition of an alkyl halide to the reaction mixture.

Examples of the base include sodium hydride. The amount of the base employed may fall within a range of 2 eq. to 5 eq. and is preferably 2 eq. to 2.5 eq.

The alkyl halide is preferably an alkyl halide formed from a lower alkyl group, particularly preferably a methyl halide. Examples of the methyl halide include methyl iodide. The amount of the alkyl halide employed may fall within a range of 2 eq. to 5 eq. and is preferably, 2 eq. to 2.5 eq.

No particular limitation is imposed on the reaction solvent, so long as the solvent does not adversely affect the reaction. The reaction solvent is preferably an aprotic solvent such as N,N-dimethylformamide or N,N-dimethylacetamide, particularly preferably N,N-dimethylacetamide.

The reaction temperature may fall within a range of -78°C to the boiling temperature of the solvent and is preferably 0°C to room temperature.

The reaction time may fall within a range of 1 hour to 24 hours. Usually, the reaction is completed within about 3 hours to about 6 hours.

### [Step b]

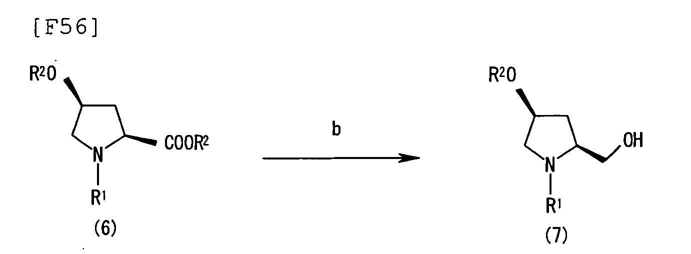

In step b, the ester group of the compound represented by formula (6) is converted through reduction into a primary hydroxy group. The reaction may be performed in a solvent in the presence of a reducing agent.

In formulas (6) and (7), R¹ and R² have the same meanings as defined above.

Examples of the reducing agent include borohydride reducing agents such as sodium borohydride, lithium borohydride, calcium borohydride, zinc borohydride, magnesium borohydride, and sodium cyanoborohydride; and aluminum hydride reducing agents such as lithium aluminum halide. Of these, borohydride reducing agents are preferred, with sodium borohydride being particularly preferred.

The amount of the reducing agent employed may fall within a range of 1.1 eq. to 5 eq. and is preferably 2 eq. to 3 eq. with respect to the amount of the compound represented by formula (6).

No particular limitation is imposed on the solvent, so long as the solvent does not adversely affect the reaction. Examples of the solvent include hydrocarbon solvents such as toluene; alcohol solvents such as methanol, ethanol, isopropanol, and t-butanol; and ethereal solvents such as diethyl ether and tetrahydrofuran. Of these, hydrocarbon solvents are preferred, with toluene being particularly preferred. When toluene is employed, the reaction is facilitated through addition of methanol in an amount 1 to 5 times eq., preferably 2 to 3 times eq., that of (6).

No particular limitation is imposed on the reaction temperature, so long as the reaction temperature does not adversely affect the reaction. The reaction temperature is preferably 0°C to 60°C, particularly preferably room temperature to 50°C.

The reaction time may fall within a range of 1 hour to 24 hours.

### [Step c]

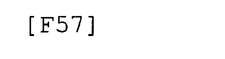

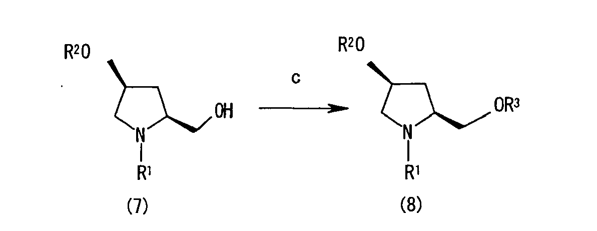

In step c, the hydroxymethyl group is converted to a substituted sulfonyloxy group; i.e., the hydroxy group is converted to a leaving group for the next substitution reaction step. The leaving group is not limited to a substituted sulfonyloxy group. No particular limitation is imposed on the leaving group, so long as the group functions as a leaving group. The leaving group may be a halogen atom, but is preferably a substituted sulfonyl group. A compound represented by formula (8) may be produced through reaction of a compound represented by formula (7) with an arylsulfonyl halide which may be substituted, or an alkylsulfonyl halide which may be substituted in the presence of a base.

In formulas (7) and (8), R¹ and R² have the same meanings as defined above. R³ represents a sulfonyl group which may be substituted, such as an arylsulfonyl group which may be substituted, or an alkylsulfonyl group which may be substituted. Examples of the arylsulfonyl group include phenylsulfonyl and C₁₋₆ alkylphenylsulfonyl. Examples of the alkylsulfonyl group include C₁₋₆ alkylsulfonyl. The substituted sulfonyl group is preferably p-toluenesulfonyl, particularly preferably methanesulfonyl.

This reaction may be performed through reaction of a compound represented by formula (7) with a substituted or non-substituted arylsulfonyl halide or alkylsulfonyl halide, preferably a substituted sulfonyl chloride, in the presence of a base.

The sulfonyl halide may be one corresponding to the sulfonyl group to be employed, and examples include p-toluenesulfonyl chloride and methanesulfonyl chloride. The sulfonyl halide is preferably employed in an amount of 1 eq. to 2.5 eq., particularly preferably 1 eq. to 1.5 eq., with respect to the amount of the compound represented by formula (7).

Examples of the base employed include organic bases such as alkyl amines (e.g., triethylamine, N,N-dimethylaniline, pyridine, 4-dimethylaminopyridine), aromatic amines, and nitrogen-containing heterocyclic compounds; and inorganic bases such as carbonates and hydrogencarbonates of alkali metals or alkaline earth metals (e.g., anhydrous potassium carbonate, anhydrous sodium carbonate, sodium hydrogencarbonate). Preferably, the base is an organic base such as triethylamine or 4-dimethylaminopyridine. Examples of particularly preferred bases include triethylamine. The base may be employed in an amount of 1 eq. to 10 eq., preferably 1 eq. to 2.5 eq., with respect to the amount of the compound (7) and in an equiamount by mole of the sulfonyl halide. When triethylamine is employed, the reaction is facilitated through addition of trimethylamine hydrochloride in an amount of 0.01 eq. to 1 eq., preferably 0.05 eq. to 0.1 eq.

No particular limitation is imposed on the reaction solvent, so long as the solvent does not adversely affect the reaction. Examples of the reaction solvent include hydrocarbon solvents such as toluene; ethereal solvents such as diethyl ether and tetrahydrofuran; chlorine-containing solvents such as methylene chloride and 1,2-dichloroethane; and nitrile solvents such as acetonitrile. Examples of preferred reaction solvents include hydrocarbon solvents such as toluene, and nitrile solvents such as acetonitrile. Examples of particularly preferred solvents include toluene.

The reaction temperature may be -78°C to the boiling temperature of the solvent, preferably 0°C to room temperature.

The reaction time may fall within a range of 5 minutes to 24 hours. Usually, the reaction is completed within about 30 minutes to about 6 hours.

### [Step d]

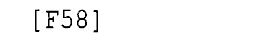

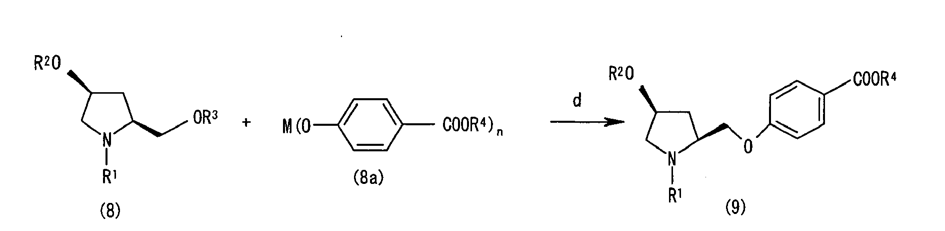

In step d, a benzoic acid unit (8a) is introduced into a compound represented by formula (8). Specifically, a compound represented by formula (8) is reacted with a compound represented by formula (8a), to thereby yield a compound represented by formula (9).

In formulas (8), (8a), and (9), R¹, R², and R³ have the same meanings as defined above. R⁴ represents an alkyl group which may be substituted, or an aralkyl group which may be substituted. Examples of the aralkyl group include phenyl-(C₁₋₆ alkyl) and (C₁₋₆ alkyl)phenyl-(C₁₋₆ alkyl). Of these, a non-substituted lower alkyl group is preferred, with methyl and ethyl being particularly preferred. M represents an alkali metal atom or an alkaline earth metal atom, and "n" is an integer of 1 or 2 (when M is an alkali metal atom, n=1; and when M is an alkaline earth metal atom, n=2).

This step of introducing a benzoic acid unit may employ a 4-hydroxybenzoic acid ester. For example, a commercially available methyl ester or ethyl ester may be employed. The benzoic acid compound is converted to a sodium, potassium, or lithium phenolate derivative or to a calcium phenolate derivative. Thereafter, the phenolate derivative may be coupled with the compound represented by formula (8). This coupling reaction is known as a common reaction for forming an aryl ether bond through use of a phenolate anion. Therefore, in the compound represented by formula (8a), a metal atom (cation) represented by M may be selected from among alkali metal atoms and alkaline earth metal atoms. The metal atom is preferably an alkali metal atom, more preferably lithium, sodium, or potassium, particularly preferably sodium or potassium.

No particular limitation is imposed on the solvent employed in the reaction, so long as the solvent does not adversely affect the reaction. Examples of the solvent include hydrocarbon solvents such as toluene; ethereal solvents such as diethyl ether and tetrahydrofuran; and aprotic polar solvents such as N,N-dimethylformamide, N-methyl-2-pyrrolidone, and dimethyl sulfoxide. Of these, for example, tetrahydrofuran and N,N-dimethylformamide are preferred. The solvent is preferably anhydrous to a generally acceptable extent.

The reaction may be performed in any of the solvents described above, through addition, to a commercially available 4-hydroxybenzoic acid ester, of an inorganic base such as a carbonate or a hydrogen carbonate of an alkali metal or an alkaline earth metal (e.g., anhydrous potassium carbonate, anhydrous sodium carbonate, sodium hydrogencarbonate) and a compound represented by formula (8). Alternatively, the reaction may be performed through treatment of a 4-hydroxybenzoic acid ester with a metal hydride to thereby prepare a phenolate (8a) and then adding a compound represented by formula (8) thereto.

The reaction temperature falls within a range of 0°C to the boiling temperature of the solvent, preferably 50°C to 120°C.

The reaction time may fall within a range of 30 minutes to 72 hours. Usually, the reaction is completed within about 20 hours to 48 hours.

This reaction is preferably performed under anhydrous conditions, in order to prevent hydrolysis of the ester group and decomposition of the sulfonyloxy compound (8).

### [Step e]

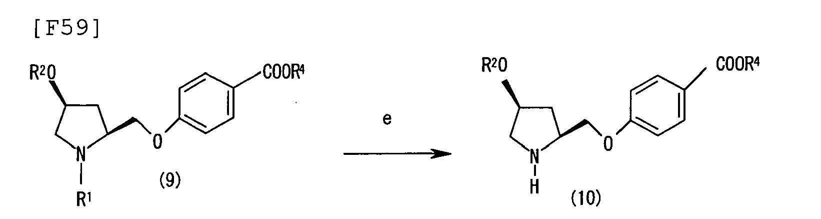

In step e, the protecting group R¹ of the nitrogen atom is removed, to thereby yield a compound represented by formula (10) as crystals in the form of a salt. This reaction may be performed through hydrolysis, catalytic hydrogenation, or a similar reaction.

In formulas (9) and (10), R¹, R², and R⁴ have the same meanings as defined above.

In step e, the protecting group located on the nitrogen atom is removed. When the protecting group in the compound represented by formula (9) contains an aromatic ring, this step of removing the protecting group is particularly desired. This is because the aromatic ring of the protecting group would otherwise be reduced in the step in which the benzoic acid aromatic ring is reduced, to thereby complicate subsequent reactions.

Step e may be carried out through a known method for removing a protecting group located on the nitrogen atom (see, for example, "Protective Groups in Organic Synthesis, edited by T. W. Greene and P. G. Wuts, John Wiley&Sons, Inc., New York, 1991"), depending on the type of the protecting group.

After the protecting group is removed, an acid is added to the solvent, and crystals that have precipitated are collected through filtration, to thereby yield a compound represented by formula (10). Here, the compound represented by formula (10) is obtained as crystals in the form of a salt. This is advantageous, since the stability of the compound represented by formula (10) is enhanced, and treatment through use of a column is eliminated.

For example, when R¹ is benzyloxycarbonyl, deprotection may be performed through catalytic hydrogenation under neutral conditions. Examples of the catalyst employed include palladium catalysts such as palladium-carbon and palladium (II) hydroxide; and platinum catalysts such as platinum dioxide. Of these, palladium-carbon is preferred.

No particular limitation is imposed on the solvent employed, so long as the solvent does not affect the reaction. Examples of the solvent include alcohol solvents such as methanol and ethanol, and ethereal solvents such as tetrahydrofuran. Of these, methanol and ethanol are preferred.

The pressure of hydrogen may fall within a range of normal pressure to 10 MPa and is preferably normal pressure to 1 MPa. Examples of the acid which is added for crystallization include carboxylic acids such as oxalic acid, tartaric acid, and trifluoroacetic acid, with oxalic acid and trifluoroacetic acid being preferred. When R² and R⁴ represent a methyl group and an ethyl group, respectively, the compound represented by formula (10) is preferably obtained in the form of an oxalic acid salt.

The solvent for crystallization may be an alcohol solvent such as methanol, ethanol, or isopropanol; or a mixed solvent of a hydrocarbon solvent (e.g., hexane) and an ethereal solvent (e.g., diethyl ether, diisopropyl ether) or an ester solvent (e.g., ethyl acetate). Of these, methanol and ethanol are preferred.

The crystallization temperature may fall within a range of 0°C to 50°C and is preferably 0°C to room temperature.

The crystallization time may fall within a range of 1 hour to 24 hours. [Step f]

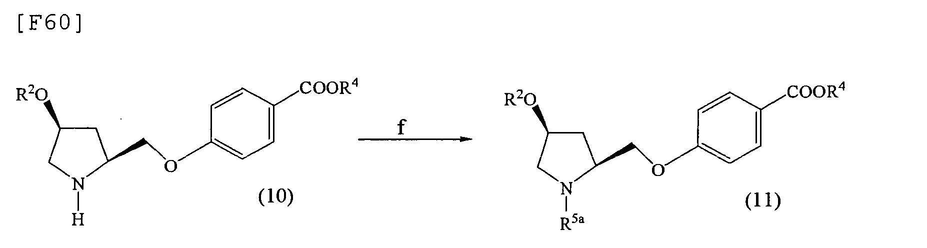

In step f, a protecting group is introduced on the nitrogen atom.

In formulas (10) and (11), R² and R⁴ have the same meanings as defined above. R^{5a} represents a protecting group for the amino group.

In order to obtain a compound (11), after completion of reaction, crystals that have precipitated in a solvent are collected through filtration.

Step f may be performed in accordance with a known method for introducing a protecting group on a nitrogen atom (see, for example, "Protective Groups in Organic Synthesis, edited by T. W. Greene and P. G. Wuts, John Wiley&Sons, Inc., New York, 1991"). The protecting group is preferably tert-butoxycarbonyl or benzyloxycarbonyl.

The solvent employed for crystallization is preferably a mixed solvent of an alcohol solvent (e.g., methanol, ethanol, isopropanol) and water. Particularly preferably, methanol or ethanol is employed.

The crystallization temperature may fall within a range of 0°C to 50°C and is preferably 0°C to room temperature.

The reaction time may fall within a range of 1 hour to 24 hours.

Steps e and f can be omitted.

### [Step g]

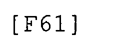

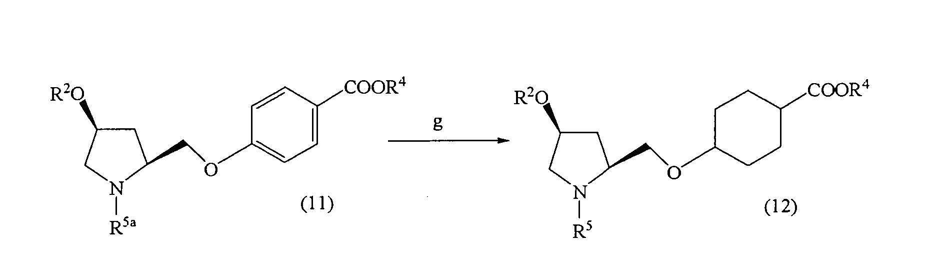

In step g, the benzene ring is converted to a cyclohexane ring through reduction.

In formulas (11) and (12), R⁵ represents a hydrogen atom or a protecting group for the amino group, and R², R⁴, and R^{5a} have the same meanings as defined above.

Methods for converting a benzene ring to a cyclohexane ring through hydrogenation have been known. Among these methods, a method which is performed under mild conditions may be employed in step g. For example, the method of W. M. Pearlman *et al.* (see "Organic Synthesis, Collective volume 5, p.670-672, John Wiley&Sons, Inc.") may be employed. This reduction reaction is highly cis-selective. Therefore, a 1,4-cis form is preferentially obtained.

The catalyst employed in the step of hydrogenating the benzene ring may be a commercially available catalyst such as palladium-carbon, platinum oxide, strontium carbonate, rhodium-alumina, or rhodium-carbon. Of these, a rhodium-alumina catalyst is most preferred.

The amount of the catalyst employed may fall within a range of 1% to 50% and is preferably 3% to 20%, with respect to the weight of the compound to be reduced.

No particular limitation is imposed on the solvent, so long as the solvent does not adversely affect the reaction. There may be employed an alcohol solvent such as methanol or ethanol, or an ethereal solvent such as tetrahydrofuran or dioxane. Of these, an alcohol solvent such as methanol or ethanol is preferred. Preferably, acetic acid or trifluoroacetic acid serving as a co-solvent is added to the solvent in an amount of 5% to 20% by volume.

The hydrogen pressure may fall within a range of normal pressure to 10 MPa and is preferably normal pressure to 1.5 MPa.

The reaction temperature may fall within a range of 0°C to 100°C and is preferably room temperature to 60°C.

The reaction time may fall within a range of 1 hour to 72 hours. Usually, the reaction is completed within about 1 hour to 48 hours.

### [Step h]

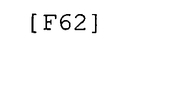

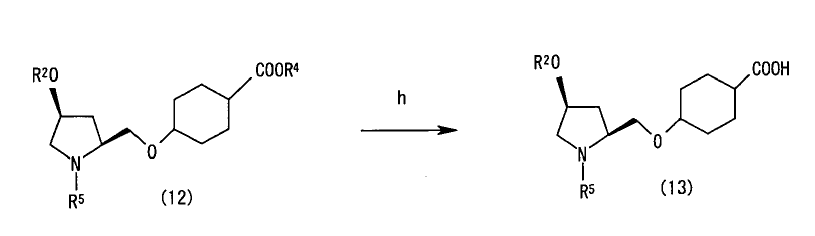

In step h, a compound represented by formula (12) is isomerized through use of a base via an enolate and then hydrolyzed, to thereby increase the ratio of a trans-form compound, which has the target stereoscopic configuration. Specifically, the compound represented by formula (12) is treated with a base in an aprotic polar solvent and then reacted with water.

In formulas (12) and (13), R², R⁴, and R⁵ have the same meanings as defined above.

Examples of the solvent employed include aprotic solvents such as N,N-dimethylformamide and N,N-dimethylacetamide; ethereal solvents such as dimethyl ether and tetrahydrofuran; and nitrile solvents such as acetonitrile. Of these, an aprotic solvent is preferred, with N,N-dimethylformamide and N,N-dimethylacetamide being particularly preferred. The reaction is facilitated through addition of an alcohol which corresponds to R⁴, in an amount of 1.5 eq. to 5 eq., preferably 2 eq. to 4 eq., with respect to the compound represented by formula (12).

Examples of the base employed include metal hydrides and metal alkoxides, with metal alkoxides being preferred. Specific examples of the metal hydride include sodium hydride, and examples of the metal alkoxide include sodium ethoxide and potassium t-butoxide. The amount of the base employed may fall within a range of 1 eq. to 4 eq. and is preferably 2 eq. to 2.5 eq.

The reaction temperature may fall within a range of 0°C to 60°C and is preferably 0°C to room temperature.

The reaction mixture is stirred at a temperature falling within the above range for 1 hour to 10 hours, and water is added to the mixture in an amount of 1 eq. to 5 eq., preferably 2 eq. to 3 eq. The resultant mixture is stirred for 5 hours to 24 hours at 0°C to 60°C, preferably 0°C to room temperature, whereby the reaction is completed.

### [Step i]

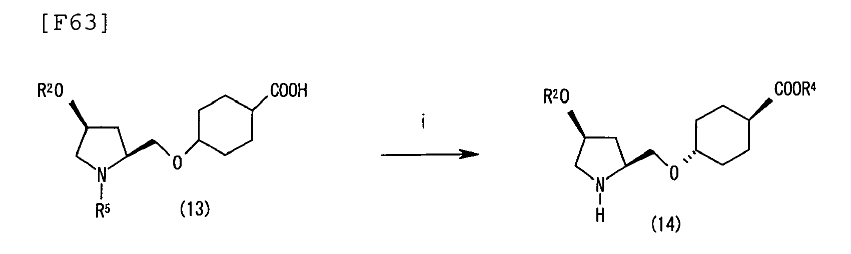

In step i, the protecting group located on the nitrogen atom is removed, while the carboxylic acid moiety is converted to an ester group, and the compound (13) is treated with an acid to thereby yield with precedence a trans-form compound as crystals. This reaction is performed through treatment of the compound (13) with an acid in the presence of an alcohol.

In formulas (13) and (14), R², R⁴, and R⁵ have the same meanings as defined above.

Prior to being used in the reaction in step i, isolation of the compound represented by formula (13) produced in step h may not be performed, or the compound may be isolated as a salt. Examples of the salt include alkali metal salts such as sodium salts, potassium salts, and lithium salts; alkaline earth metal salts such as magnesium salts and calcium salts; and organic amine salts such as triethylamine salts, N-methylglucamine salts, N-benzylethanolamine salts, ethanolamine salts, tert-butylamine salts, tris(hydroxymethyl)aminomethane salts, and cyclohexylamine salts. Particularly, cyclohexylamine salts are preferred.

The acid employed for removing the protecting group may be selected in accordance with a known method for removing a protecting group located on a nitrogen atom. Examples of the acid include inorganic acids and organic acids, including hydrochloric acid, sulfuric acid, commercially available 1N HCl-ethanol, 4N HCl-dioxane, (-) camphorsulfonic acid, camphorsulfonic acid, p-toluenesulfonic acid, and trifluoroacetic acid. When an inorganic acid is employed, the solvent may be a carbon solvent such as toluene or methyl ethyl ketone, or an alcohol solvent such as methanol or ethanol. Of these acids, (-) camphorsulfonic acid and 1N HCl-ethanol are preferred.

The acid employed for producing a trans-form salt is preferably (-) camphorsulfonic acid, (±) camphorsulfonic acid, or (-) tartaric acid, particularly preferably (-) camphorsulfonic acid. The acid is preferably added in an amount of 0.5 eq. to 2 eq., particularly preferably 0.7 eq. to 1 eq.

Preferably, the same acid is employed for removing the protecting group located on the nitrogen atom and for producing a trans-form salt. Particularly, (-) camphorsulfonic acid is preferred. In this case, single addition of an acid enables removal of the protecting group, conversion to an ester group, and production of a trans-form compound.

The reaction temperature may fall within a range of room temperature to the boiling temperature of the solvent, and is preferably 50°C to the boiling temperature of the solvent.

The reaction time falls within a range of 1 hour to 24 hours.

In the case where different acids are employed for removing the protecting group located on the nitrogen atom and for producing a trans-form salt, the compound represented by formula (14) is obtained by reacting the compound represented by formula (13) with an acid for removing the protecting group on the nitrogen atom, neutralizing the product with an inorganic base, adding, in a solvent, an acid for producing a trans-form salt to thereby allow crystals to precipitate, and collecting the crystals through filtration.

The inorganic base employed for neutralization is preferably a carbonate or a hydrogencarbonate of an alkali metal or an alkaline earth metal, such as sodium hydrogencarbonate, potassium hydrogencarbonate, sodium carbonate, or potassium carbonate.

Examples of the solvent employed include hydrocarbon solvents such as toluene; ketone solvents such as acetone and methyl ethyl ketone; ester solvents such as ethyl acetate; and alcohol solvents such as isopropyl alcohol. Of these, methyl ethyl ketone and ethyl acetate are preferred.

The crystallization temperature may fall within a range of 0°C to the boiling temperature of the solvent, and is preferably 0°C to 50°C.

The crystallization time may fall within a range of 1 hour to 24 hours.

When R² and R⁴ are a methyl group and an ethyl group, respectively, in the compound represented by the above formula (14), the compound is obtained as a (-) camphorsulfonic acid salt, whereby the target trans-form is obtained with precedence so that an isomer of interest can be isolated.

### [Steps j and k]

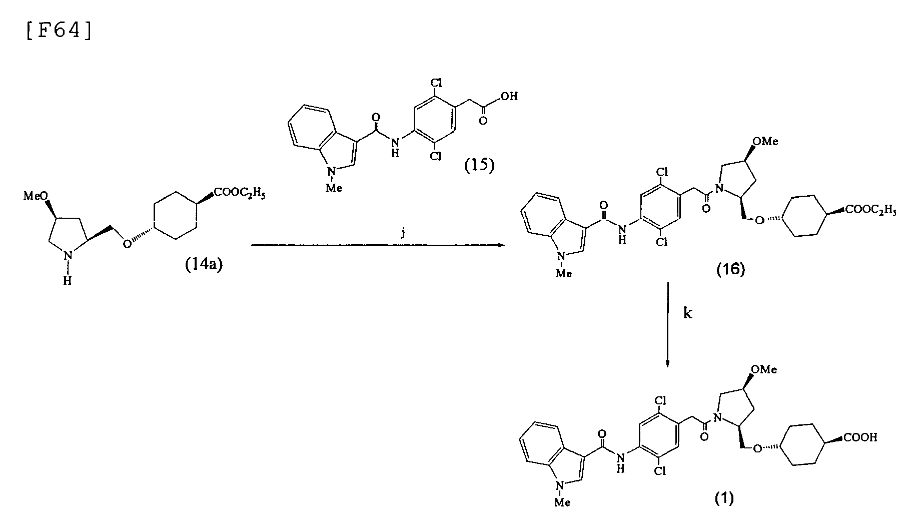

The thus-produced compound represented by formula (14) is condensed with a carboxylic acid through a known method. When R² represents a methyl group, and R⁴ represents an ethyl group in the compound represented by formula (14) (formula (14a)), the compound may be condensed with a compound represented by formula (15), to thereby yield a compound represented by formula (16) (wherein R² represents a methyl group, and R⁴ represents an ethyl group) (step j). The compound represented by formula (15) may be produced through a method described in WO2002/053534.

The step of converting a compound represented by formula (16) to a compound represented by formula (1) may be performed through a known method for cleaving an ester moiety (see "Protective Groups in Organic Synthesis, edited by T. W. Greene and P. G. Wuts, John Wiley&Sons, Inc., New York, 1991"), to thereby yield a free carboxylic acid (step k).

### [Step 1]

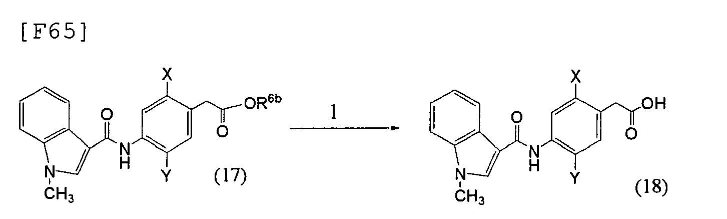

In step 1, an ester compound represented by formula (17) is hydrolyzed to thereby yield a compound represented by formula (18). In formula (17), R^{6b} represents a linear or branched lower alkyl group which may be substituted, or an aralkyl group which may be substituted. Examples of the lower alkyl group include a C1-C6 alkyl group. Examples of the aralkyl group include a phenyl(C₁₋₆ alkyl) group. The substituent which may be bound to the alkyl group or the aralkyl group may be 1 to 3 groups selected from halogen atoms, C1-C6 alkoxy groups, and the like. Preferred examples of R^{6b} include methyl, ethyl, tert-butyl, benzyl, and 4-methoxybenzyl, with methyl and ethyl being particularly preferred. X represents a hydrogen atom or a halogen atom, and Y represents a halogen atom or a lower alkoxy group. Examples of the lower alkoxy group include C1-C6 alkoxy groups. Preferably, X and Y each independently represent a chlorine atom or a fluorine atom. More preferably, both of X and Y represent a chlorine atom.

Step 1 is a widely used process for hydrolyzing an ester to thereby produce a free carboxylic acid, and may be performed through a common method for converting an alkoxycarbonyl group to a carboxylic acid (see "Protective Groups in Organic Synthesis, edited by T. W. Greene and P. G. Wuts, John Wiley&Sons, Inc., New York, 1991").

Examples of the solvent employed include alcohol solvents such as methanol, ethanol, and isopropyl alcohol; and aprotic polar solvents such as dimethyl sulfoxide, N,N-dimethylformamide, N,N-dimethylacetamide, and acetonitrile. Of these, methanol is preferred.

The hydrolyzing agent may be an acid or an alkali, such as a hydroxide of an alkali metal such as sodium, potassium, or lithium or of an alkaline earth metal such as magnesium or calcium. Of these, sodium hydroxide is preferred.

The reaction temperature preferably falls within a range of room temperature to the boiling temperature of the solvent.

The reaction time may fall within a range of 30 minutes to 5 hours. Usually, the reaction is completed within 1 hour to 2 hours.

### [step m]

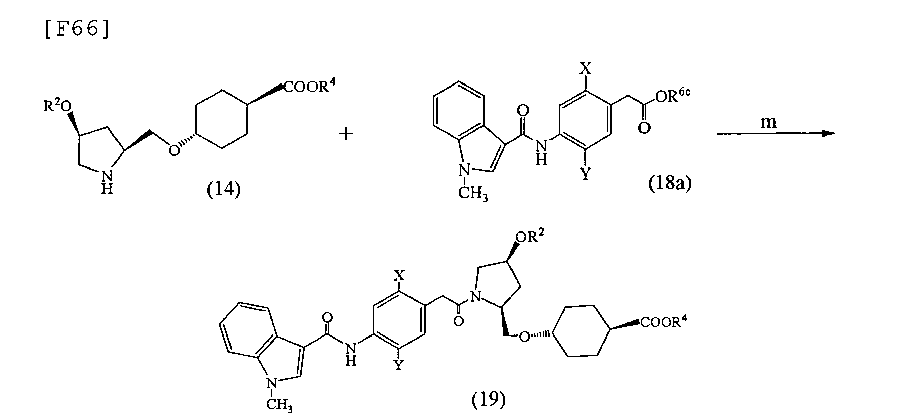

In step m, a compound represented by formula (14) is condensed with a compound represented by formula (18a) to thereby yield a compound represented by formula (19). The compound represented by formula (18a) is any one of the compound represented by formula (17) and the compound represented by formula (18). In the above formulas, R², R⁴, X, and Y have the same meanings as defined above. R^{6c} represents a hydrogen atom or R^{6b}. When -OR^{6c} of the compound represented by formula (18a) is an ester group, the ester group is preferably an activated ester.

Step m may employ a known amidation reaction.

No particular limitation is imposed on the solvent employed, so long as the solvent does not adversely affect the reaction. Examples of the solvent include halogenated hydrocarbon solvents such as methylene chloride, hydrocarbon solvents such as toluene, ethereal solvents such as tetrahydrofuran, and aprotic polar solvents such as dimethyl sulfoxide, N,N-dimethylformamide, N,N-dimethylacetamide, and acetonitrile. Acetonitrile is particularly preferred.

The reaction of step m may be performed in any of the above solvents through use of a condensing agent selected from 1-ethyl-3-(3-dimethylaminopropyl)carbodiimide hydrochloride, N,N-dicyclohexylcarbodiimide, N,N-carbonyldiimidazole, and similar compounds. Preferably, the reaction is performed in N,N-dimethylformamide through addition of 1-ethyl-3-(3-dimethylaminopropyl)carbodiimide hydrochloride or N,N-dicyclohexylcarbodiimide in an amount of 1 eq. to 3 eq., preferably 1 eq. to 1.5 eq.

The reaction of step m may be performed in the presence of an organic amine base such as triethylamine or N,N-dimethylaminopyridine. The organic amine base is preferably added in an amount of 1 eq. to 4 eq., particularly preferably 2 eq. to 2.5 eq.

The reaction of step m may be performed in the presence of an activated esterification reagent such as 1-hydroxybenzotriazole. The reagent is added in an amount of 0.5 eq. to 3 eq., preferably 1 eq. to 1.5 eq.

The reaction temperature falls within a range of room temperature to 50°C, preferably room temperature. The reaction time is 2 to 24 hours. Usually, the reaction is completed within about 4 to about 6 hours. [Step n]

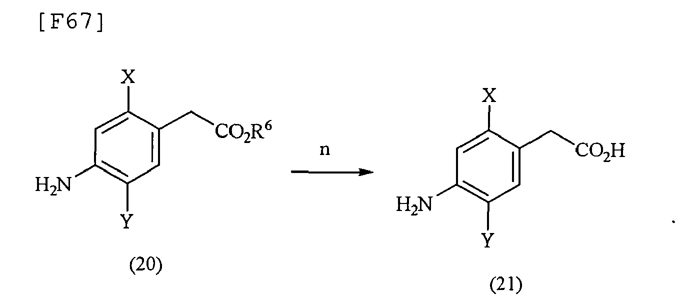

In step n, an ester compound represented by formula (20) is hydrolyzed to thereby yield a carboxylic acid. In formula (20), R⁶ represents a hydrogen atom, a linear or branched lower alkyl group which may be substituted, or an aralkyl group which may be substituted. X and Y have the same meanings as defined above.

Similar to step 1, step n is performed through a common hydrolysis method.

### [Step o]

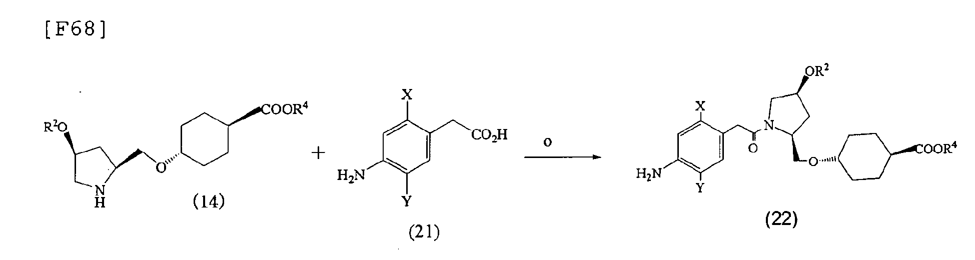

In step o, a carboxylic acid represented by formula (21) is condensed with a compound represented by formula (14). In the above formulas, R², R⁴, X, and Y have the same meanings as defined above.

Step o may employ a common condensation reaction.

No particular limitation is imposed on the solvent employed, so long as the solvent does not adversely affect the reaction. Examples of the solvent include halogenated hydrocarbon solvents such as methylene chloride, hydrocarbon solvents such as toluene, ethereal solvents such as tetrahydrofuran, and aprotic polar solvents such as acetonitrile, N,N-dimethylformamide, and N-methyl-2-pyrrolidone. Acetonitrile is preferred.

The reaction of step o may be performed in any of the above solvent through use of a condensing agent selected from 1-ethyl-3-(3-dimethylaminopropyl)carbodiimide hydrochloride, N,N-dicyclohexylcarbodiimide, N,N-carbonyldiimidazole, and similar compounds. Preferably, the reaction is performed in N,N-dimethylformamide through addition of 1-ethyl-3-(3-dimethylaminopropyl)carbodiimide hydrochloride or N,N-dicyclohexylcarbodiimide in an amount of 1 eq. to 3 eq., preferably 1 eq. to 1.5 eq.

The reaction of step o may be performed in the presence of an organic amine base such as triethylamine or N,N-dimethylaminopyridine. The organic amine base is preferably added in an amount of 1 eq. to 4 eq., particularly preferably 2 eq. to 2.5 eq.

The reaction of step o may be performed in the presence of an activated esterification reagent such as 1-hydroxybenzotriazole. The reagent is added in an amount of 0.5 eq. to 3 eq., preferably 1 eq. to 1.5 eq.

The reaction temperature falls within a range of room temperature to 50°C, preferably room temperature. The reaction time is 2 to 24 hours. Usually, the reaction is completed within about 4 to about 6 hours.

### [Step p]

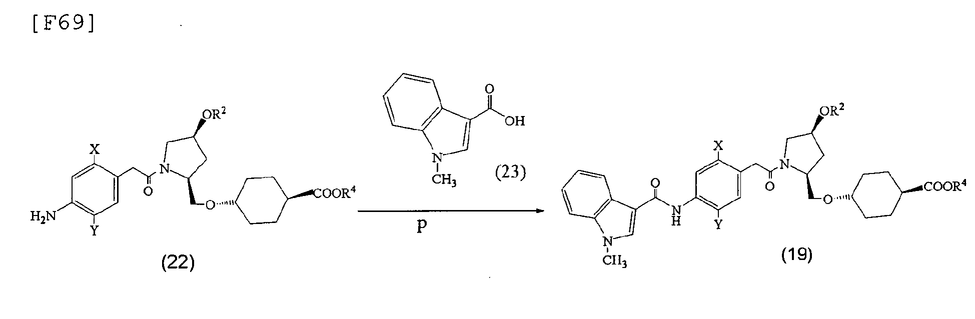

In step p, a compound represented by formula (22) is caused to form an amide linkage, to thereby yield a compound represented by formula (19). In the above formulas, R², R⁴, X, and Y have the same meanings as defined above.

Examples of the reaction solvent include chlorine-containing solvents such as methylene chloride and 1,2-dichloroethane, hydrocarbon solvents such as toluene and benzene, ethereal solvents such as tetrahydrofuran, ester solvents such as ethyl acetate, and acetonitrile. Preferably, the solvent is a chlorine-containing solvent such as methylene chloride or 1,2-dichloroethane, a hydrocarbon solvent such as toluene or benzene, or acetonitrile. Particularly preferably, the solvent is 1,2-dichloroethane, toluene, or acetonitrile.

The reaction temperature preferably falls within a range of room temperature to the boiling temperature of the solvent. The reaction time may fall within a range of 5 to 20 hours. Usually, the reaction is completed within about 10 hours.

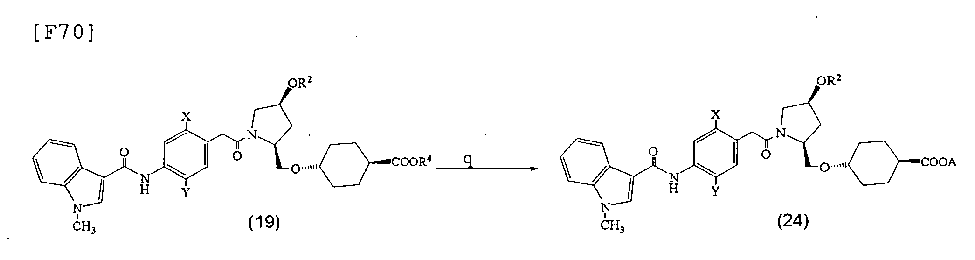

In this step, a compound represented by formula (19) is obtained as a slat form. In the above formulas, R², R⁴, X, and Y have the same meanings as defined above.

Examples of A include a hydrogen atom; alkali metals such as sodium, potassium, and lithium; alkaline earth metals such as magnesium and calcium; and organic amines such as triethylamine, N-methylglucamine, N-benzylethanolamine, ethanolamine, tert-butylamine, tris(hydroxymethyl)aminomethane, and cyclohexylamine. The compound represented by formula (24) may be isolated as a hydrate.

Examples of the solvent employed include alcohol solvents such as methanol, ethanol, and isopropyl alcohol, and aprotic polar solvents such as dimethyl sulfoxide, N,N-dimethylformamide, N,N-dimethylacetamide, and acetonitrile. Preferably, the solvent is isopropyl alcohol.

Examples of the hydrolyzing agent include hydroxides of alkali metals such as sodium, potassium, and lithium; and hydroxides of alkaline earth metals such as magnesium and calcium. Of these, sodium hydroxide is preferred.

The reaction temperature preferably falls within a range of room temperature to the boiling temperature of the solvent.

The reaction time may fall within a range of 30 minutes to 5 hours. Usually, the reaction is completed within about 2 hours.

Purification of the compound represented by formula (24) may be performed through combined use of any of washing, extraction, recrystallization, chromatography, and other means, as desired.

Examples of the solvent employed in recrystallization include alcohol solvents such as methanol, ethanol, and isopropyl alcohol; aprotic polar solvents such as dimethyl sulfoxide, N,N-dimethylformamide, N,N-dimethylacetamide, and acetonitrile; and water-soluble hydrocarbon solvents such as methyl butyl ketone and acetone. Acetone is preferred. Hydrous percentage of solvent may fall within a range of 1 to 20%, preferably 10%.

Recrystallization may be performed through dissolution of crude crystals in any of the above solvents, and carbon-treatment with activated carbon. The amount of activated carbon added is 0.01 to 0.5 times, preferably 0.05 times, with respect to the weight of the crude crystals.

The stirring temperature may fall within a range of 10 to 40°C and is preferably room temperature (15 to 25°C). The stirring time may fall within a range of 3 to 40 hours and is preferably 20 to 30 hours.

### [Examples]

The present invention will next be described in detail by way of Examples, which should not be construed as limiting the invention thereto.

### [Example 1]

### (4R)-Methoxy-(2S)-proline methyl ester-1-carboxylic acid benzyl ester (in the following formula, Z represents a benzyloxycarbonyl group)

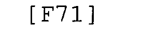

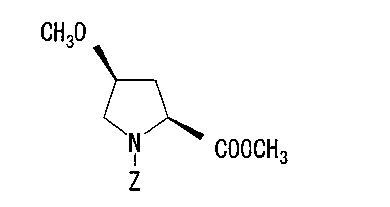

(1) 1-Benzyloxycarbonyl-(4R)-methoxy-(2S)-proline (150 g, 0.57 mol) was dissolved in dimethylformamide (DMF) (1,000 mL), and NaH (47.5 g, 2.1 eq.) was added to the solution at an internal temperature of 30°C to 50°C. At the same temperature, the mixture was stirred for about 30 minutes, and MeI (77.0 mL, 2.2 eq.) was added dropwise to the reaction mixture, followed by stirring for 4 hours. Water was added to the reaction mixture, and the resultant mixture was extracted twice with toluene. The combined organic layer was washed twice with water and then concentrated until the volume of toluene was reduced by approximately one-half.
   ¹H-NMR(CDCl₃)δ: 2.04-2.37 (m, 2H), 3.26(s, 3H), 3.54-3.76(m, 5H), 3.93-3.96(m, 1H), 4.41-4.51(m, 1H), 5.06-5.21(m, 2H), 7.27-7.38(m, 5H)
(2) 1-Benzyloxycarbonyl-(4R)-methoxy-(2S)-proline (100.0 g, 377.0 mM, 1.0 Meq.) was dissolved in DMAc (1,000 mL, 10 v/w), and the solution was cooled to an internal temperature of 5°C. MeI (93.4 mL, 1508.3 mM, 4.0 Meq., 0.93 v/w) was added dropwise to the cooled solution, and 28% NaOH aq. (110 mL, 979.2 mM, 2.6 Meq., 1.1 v/w) was added dropwise to the mixture, followed by stirring for 8 hours at an internal temperature of 5°C. Thereafter, the reaction mixture was diluted with toluene (1,000 mL, 10 v/w), and 1N HCl aq. was added dropwise to the resultant mixture to thereby adjust the pH to 7. The resultant mixture was partitioned to an organic layer and an aqueous layer, and the aqueous layer was extracted with toluene (1,000 mL, 10 v/w). The organic layers were combined together, washed with water (600 mL, 6 v/w), and dried over MgSO₄, followed by filtration. Thereafter, the solvent was removed under reduced pressure, to thereby yield a colorless oil (134.4 g) (containing (4R)-methoxy-(2S)-proline methyl ester-1-carboxylic acid benzyl ester in an amount of 69.6%, yield 85.0%) (containing (4R)-methoxy-(2S)-hydroxymethylpyrrolidine-1-carboxylic acid benzyl ester 3.1%, yield 4.0%).

### [Example 2]

### (4R)-Methoxy-(2S)-hydroxymethylpyrrolidine-1-carboxylic acid benzyl ester (in the following formula, Z represents a benzyloxycarbonyl group)

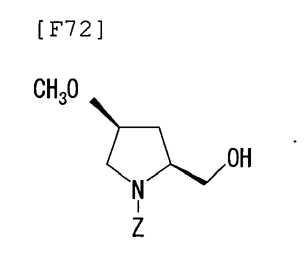

NaBH₄ (42.8 g, 2 eq.) was added to (the above solution of) (4R)-methoxy-(2S)-proline methyl ester-1-carboxylic acid benzyl ester in toluene at room temperature, and MeOH (274 mL, 12 eq.) was added dropwise to the mixture at an internal temperature of 25 to 45°C, followed by stirring for 3 hours. Water was added to the reaction mixture, and the resultant mixture was partitioned. The aqueous layer was extracted with toluene, and the organic layers were combined and then washed twice with water, and then concentrated until the volume of toluene was reduced by approximately one-half.
¹H-NMR(CDCl₃)δ: 1.75-1.88(m, 2H), 2.17-2.25(m, 1H)3.27-3.35(m, 3H), 3.52-3.70(m, 2H), 3.72-3.94(m, 3H), 4.05-4.15(m, 1H), 5.10-5.18(m, 2H), 7.27-7.40(m, 5H)

### [Example 3]

### (4R)-Methoxy-(2S)-(p-toluenesulfonyloxymethyl)pyrrolidine-1-carboxylic acid benzyl ester (in the following formula, Z represents a benzyloxycarbonyl group, and Ts represents a p-toluenesulfonyl group)

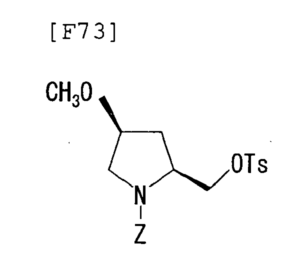

(1) Triethylamine (117.4 mL, 1.5 eq.) and trimethylamine hydrochloride (5.4 g, 0.1 eq.) were added to (the above solution of) (4R)-methoxy-(2S)-hydroxymethylpyrrolidine-1-carboxylic acid benzyl ester in toluene, and tosyl chloride (107.7 g, 1 eq.) was added to the mixture under cooling with ice, followed by stirring for 5 hours. The reaction mixture was washed with water and aqueous sodium bicarbonate, and then concentrated.
   ¹H-NMR(CDCl₃)δ: 1.92-1.99(m, 1H), 2.15-2.37(m, 1H)2.41and2.44(Sx2, 3H), 3.18-3.27(m, 3H), 3.42-3.55(m, 2H), 3.80-3.91(m, 1H), 3.92-4.28(m, 3H), 5.05-5.09(m, 2H), 7.27-7.37(m, 7H), 7.68-7.70(d, J=8.0, 1H), 7.79-7.81(d, J=8.0, 1H)
(2) (4R)-Methoxy-(2S)-hydroxymethylpyrrolidine-1-carboxylic acid benzyl ester (14.8 g, 55.6 mM, 1.0 Meq.) was dissolved in acetonitrile (120 mL, 8 v/w). Dimethylaminopyridine (DMAP) (815.0 mg, 6.7 mM, 0.12 Meq., 0.055 w/w), Et₃N (15.4 mL, 111.1 mM, 2.0 Meq., 1.05 v/w), and TsCl (15.9 g, 83.4 mM, 1.5 Meq., 1.08 w/w) were added to the solution, and the mixture was stirred for 24 hours at an external temperature of 50°C. After completion of reaction, 5N NaOH (20 mL, 1.3 v/w) was added to the reaction mixture, and the resultant mixture was stirred for 15 minutes. The reaction mixture was partitioned through use of toluene (120 mL, 8 v/w), and the aqueous layer was extracted with toluene (120 mL, 8 v/w).
   The organic layers were combined together, washed with 1N HCl (120 mL, 8 v/w), and dried over MgSO₄, followed by filtration. The solvent was removed under reduced pressure, to thereby give a brown oil (21.74 g) (containing (4R)-methoxy-(2S)-(p-toluenesulfonyloxymethyl)pyrrolidine-1-carboxylic acid benzyl ester in an amount of 85.0%, 79.3%).

### [Example 4]

### 4-((4S)-Hydroxy-(2S)-pyrrolidinylmethoxy)benzoic acid ethyl ester-1-carboxylic acid benzyl ester (in the following folmula, Z represents a benzyloxycarbonyl group)

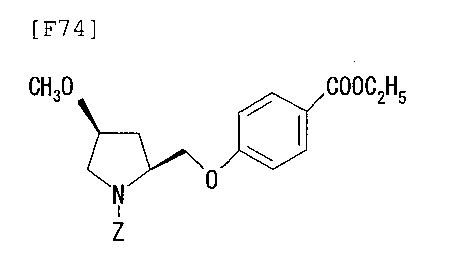

The residue obtained through concentration of the organic layer containing (4R)-methoxy-(2S)-(p-toluenesulfonyloxymethyl)pyrrolidine-1-carboxylic acid benzyl ester was dissolved in N,N-dimethylformamide (1,000 mL). Potassium carbonate (117.2 g, 1.5 eq.) and ethyl 4-hydroxybenzoate (94.0 g, 1 eq.) were added to the solution, and the mixture was stirred overnight at an internal temperature of 60 to 80°C. Subsequently, water was added to the reaction mixture, and the resultant mixture was extracted twice with toluene. The organic layers were combined, washed with 10% aqueous potassium carbonate and 1N hydrochloric acid, and concentrated.
¹H-NMR(CDCl₃)δ:
1.38 (t,J=7.2Hz,3H), 2.07 (m,1H), 2.32 (brd,J=14.4Hz,1H), 3.29 (s,3H ,OMe), 3.51-3.69 (m,2H), 3.97 (m,1H), 4.04 (m,1H), 4.17-4.47 (series of m,including q at δ
4.34, J=7.2Hz, total 4H), 5.16 (m,2H), 6.81 (d,J=8.8Hz,1H), 6.97 (d,J= 8.8Hz,1H), 7.37 (m,5H), 7.87 (d,J=8.8Hz,1H), 7.98 (d,J=8.8Hz,1H).
MS (ESI); m/z: 414 (M⁺+1).

### [Example 5]

### 4-((4S)-Methoxy-(2S)-pyrrolidinylmethoxy)benzoic acid ethyl ester·oxalic acid salt

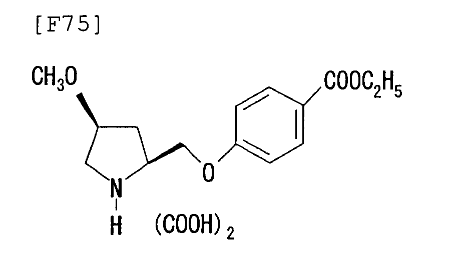

The residue obtained through concentration of the organic layer containing 4-((4S)-hydroxy-(2S)-pyrrolidinylmethoxy)benzoic acid ethyl ester-1-carboxylic acid benzyl ester was dissolved in ethanol, and the solvent was partially removed for an azeotrope. Palladium carbon (15 g) was added to the ethanol solution (0.1 w/w%), and hydrogen gas was introduced to the mixture, followed by stirring for 5 hours. Subsequently, palladium carbon was removed through filtration, and the filtrate was condensed until the volume was reduced by approximately one-half. Oxalic acid was added to the resultant ethanol solution, and the mixture was stirred for 3 hours under cooling with ice. Thereafter, the crystals were collected through filtration, to thereby give 125.7 g of 4-((4S)-methoxy-(2S)-pyrrolidinylmethoxy)benzoic acid ethyl ester·oxalic acid salt (60.2%, with respect to (4R)-methoxy-(2S)-prolinemethylester-1-carboxylic acid benzyl ester).
¹H-NMR(CDCl₃)δ: 1.36-1.39(t, J=7.2, 3H), 2.05-2.10(m, 1H), 2.51-2.58(m, 1H), 3.34-3.43(m, 1H), 3.37(s, 3H), 3.61-3.64(d, J=12.8, 3H), 4.19-4.47(m, 6H), 7.07-7.09(d, J=9.2, 2H), 8.01-8.02(d, J=8.8, 2H)

### [Example 6]

### 4-(N-t-Butoxycarbonyl-(4S)-methoxy-(2S)-pyrrolidinylmethoxy)benzoic acid ethyl ester (in the following formula, Boc represents a tert-butoxycarbonyl group)

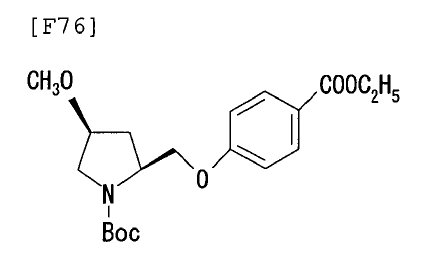

4-((4S)-Methoxy-(2S)-pyrrolidinylmethoxy)benzoic acid ethyl ester·oxalic acid salt (100 g, 0.38 mol) was dissolved in methanol (400 mL) and water (400 mL). Sodium bicarbonate (56.8 g, 2.5 eq.) was added to the solution, and the mixture was stirred for 30 minutes. The formed crystals were removed through filtration, and tert-butyl dicarbonate (68.4 mL, 1.1 eq.) was added dropwise to the filtrate. The mixture was stirred overnight, and again stirred for 4 hours under cooling with ice. The crystals that precipitated were collected through filtration, to thereby give 4-(N-t-butoxycarbonyl-(4S)-methoxy-(2S)-pyrrolidinylmethoxy)benzoic acid ethyl ester (94.4 g, 91.9%).
¹H-NMR(CDCl₃)δ: 1.36-1.39(t, J=7.2, 3H), 1.49(s, 9H), 2.00-2.15(m, 1H), 2.28-2.31(dd, J=1.6, 14.01H), 3.33(s, 3H), 3.47-3.55(m, 2H), 3.94-4.17(m, 2H), 4.25-4.37(m, 4H), 6.90-7.00(m, 2H), 7.97-7.99(d, J=8.8, 2H)

### [Example 7]

### 4-(N-t-Butoxycarbonyl-(4S)-methoxy-(2S)-pyrrolidinylmethoxy)cyclohexanecarboxylic acid ethyl ester (in the following formula, Boc represents a tert-butoxycarbonyl group)

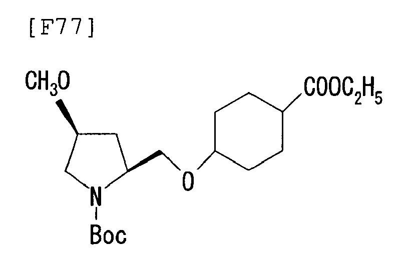

(1) 4-(N-t-Butoxycarbonyl-(4S)-methoxy-(2S)-pyrrolidinylmethoxy)benzoic acid ethyl ester (9 g, 0.024 mol) was dissolved in ethanol (45 mL) and acetic acid (1.4 mL). Rh/Al₂O₃ catalyst (1.8 g) was added to the solution, and H₂ gas was introduced to the mixture. The mixture was stirred for 1 hour at a pressure of 2 MPa. The Rh/Al₂O₃ catalyst was removed through filtration, and the reaction mixture was concentrated.
   ¹H-NMR(CDCl₃)δ: 1.24 and 1.25(t,J=7.2Hz,total 3H),1.28-2.38 (series of m, including s at δ 1.46,total 20H), 3.14-4.16 (series of m, including s at δ 3.30,total 12H).
   LC-MS; m/z: 286(M⁺-Boc+1),408(M⁺+Na).
(2) 4-(N-t-Butoxycarbonyl-(4S)-methoxy-(2S)-pyrrolidinylmethoxy)benzoic acid ethyl ester (30 g, 79.1 mol) was dissolved in ethanol (120 mL) and acetic acid (6 mL). Rh/Al₂O₃ catalyst (3 g) was added to the solution, and H₂ gas was introduced to the mixture. The mixture was stirred for 3 hours at a pressure of 2 MPa. After completion of reaction, the Rh/Al₂O₃ catalyst was removed through filtration, and the filtrate was concentrated. Subsequently, aqueous sodium bicarbonate was added to the concentrated filtrate, and the mixture was extracted with toluene and washed with water. The solvent was removed. The residue obtained through concnentration was employed in the next step without further treatment.

### [Example 8]

### 4-(N-t-Butoxycarbonyl-(4S)-methoxy-(2S)-pyrrolidinylmethoxy)cyclohexanecarboxylic acid (in the following formula, Boc represents a tert-butoxycarbonyl group)

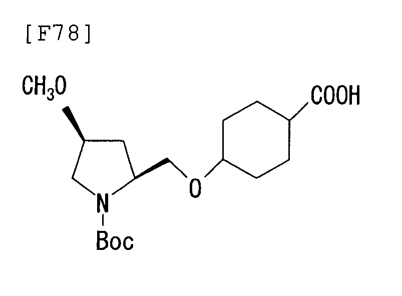

The residue 4-(N-t-butoxycarbonyl-(4S)-methoxy-(2S)-pyrrolidinylmethoxy)cyclohexanecarboxylic acid ethyl ester was dissolved in N,N-dimethylacetamide. The solution was cooled to an internal temperature of 10°C or lowern and NaH (2.1 g, 52.5 mmol) and ethanol (2.8 mL, 47.5 mmol) were added to the solution, followed by stirring for 1 hour at room temperature. The reaction mixture was cooled with ice, and water (0.5 mL) was added to the reaction mixture. The resultant mixture was stirred overnight at room temperature. 2N HCl was added to the reaction mixture, and the resultant mixture was extracted twice with toluene and then washed with water. The solvent was removed.
¹H-NMR(400MHz, CDCl₃)δ: 1.13-1.32 (2H,m), 1.46 (9H,s,t-Bu), 1.49-1.65 (3H,m), 2.05-2.29 (7H,m) 3.24-3.26 (1H,m), 3.30 (3H,s,OMe), 3.34-3.96 (6H,m)

### [Example 9]

### trans-4-((4S)-Methoxy-(2S)-pyrrolidinylmethoxy)cyclohexanecarboxylic acid ethyl ester·() camphorsulfonic acid salt

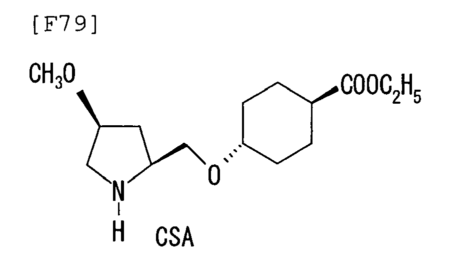

The residue 4-(N-t-butoxycarbonyl-(4S)-methoxy-(2S)-pyrrolidinylmethoxy)cyclohexanecarboxylic acid was dissolved in 1N HCl-EtOH, and the solution was stirred overnight at 50°C. The solvent was removed, and the residue was dissolved in ethyl acetate. Sodium bicarbonate was added to the solution, and the mixture was stirred for 1 hour at room temperature. The formed inorganic salt was removed through filtration, and the filtrate was concentrated. The residue was dissolved in methyl isobutyl ketone, and then formed into a salt with (-) camphorsulfonic acid. The crystals that precipitated were collected through filtration and then dried, to thereby give trans-4-((4S)-methoxy-(2S)-pyrrolidinylmethoxy)cyclohexanecarboxylic acid ethyl ester·(-) camphorsulfonic acid salt (9.3 g, 68%).
¹H-NMR (400 MHz,CDCl₃) δ: 1.21-1.27(5H,m),1.41-1.51 (3H,m), 1.99-2.12 (5H,m), 2.23-2.25 (1H,m), 2.91-2.96(1H,m),3.09-3.12(2H,m),3.22-3.28(5H,m),3.45-3.54(2H,m),3.90-3.93(1H,m),4.08-4.14(2H,m)

### [Example 10]

### 4-(N-t-Butoxycarbonyl-(4S)-methoxy-(2S)-pyrrolidinylmethoxy)cyclohexanecarboxylic acid cyclohexylamine salt

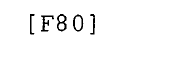

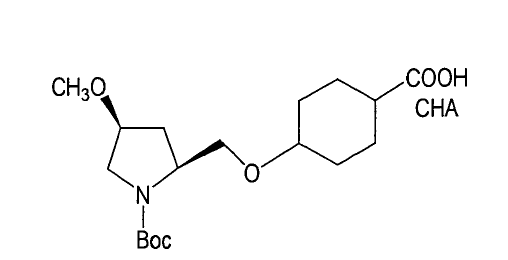

The residue obtained in Example 7 (2) through concentration of a mixture containing 4-(N-t-butoxycarbonyl-(4S)-methoxy-(2S)-pyrrolidinylmethoxy)cyclohexanecarboxylic acid ethyl ester was dissolved in N,N-dimethylacetamide (210 mL). Ethanol (181.8 mmol) was added to the solution, and the mixture was cooled to 10°C or lower. Potassium-t-butoxide (166.0 mmol) was added to the mixture, and the resultant mixture was stirred for 15 minutes. After completion of reaction, water (94.8 mmol) was added to the reaction mixture, and the resultant mixture was stirred overnight at the same temperature. After completion of reaction, 2N hydrochloric acid was added at a temperature of 10°C or lower, and the mixture was extracted twice with toluene. The organic layers were combined and then washed with water. The organic layer was concentrated, and the residue was dissolved in ethyl acetate (360 mL). Cyclohexylamine (79.1 mmol) was added to the solution, and the mixture was stirred for 6 hours at room temperature. The crystals that precipitated were collected through filtration, washed with ethyl acetate, and dried under reduced pressure at 40°C, to thereby give 4-(N-t-butoxycarbonyl-(4S)-methoxy-(2S)-pyrrolidinylmethoxy)cyclohexanecarboxylic acid cyclohexylamine salt (32.0 g, 88.6%) as white crystals.
¹H-NMR(400MHz, CDCl₃)δ: 1.06-1.45(10H, m), 1.46(9H, s, tBu), 1.55-1.70(1H, m), 1.70-1.88(3H,m), 1.90-2.12(8H,m), 2.15-2.28(1H,m), 2.76-2.90(1H,m), 3.12-3.26(1H, m), 3.30(3H, s, OMe), 3.31-4.20(6H,m)

### [Example 11]

### trans-4-((4S)-Methoxy-(2S)-pyrrolidinylmethoxy)cyclohexanecarboxylic acid ethyl ester·(-) camphorsulfonic acid salt

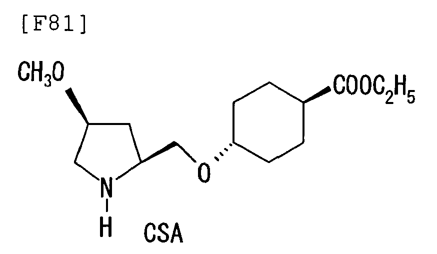

Toluene and 2N hydrochloric acid were added to 4-(N-t-butoxycarbonyl-(4S)-methoxy-(2S)-pyrrolidinylmethoxy)cyclohexanecarboxylic acid cyclohexylamine salt (15.9 g, 34.8 mmol), and the mixture was stirred and then separated. The separatd organic later was washed with 2N hydrochloric acid and water, and was concentrated. Ethanol was added to the residue for an azeotrope. Ethanol (48 mL) was added to the product, and (-) camphorsulfonic acid (41.9 mmol) was added thereto, followed by stirring for 5 hours at 70°C. After completion of reaction, the reaction mixture was concentrated. Ethyl acetate (48 mL) was added to the residue, and the mixture was stirred overnight at room temperature. The reaction mixture was cooled to 10°C or lower and then stirred for 3 hours.
The crystals that precipitated were collected through filtration and then washed with cooled ethyl acetate. The crystals were dried under reduced pressure at 40°C, to thereby give crude, white crystals (11.3 g, 62.3%).

Ethanol (15 mL) was added to the crude crystals (5.0 g, 9.7 mmol), and the mixture was heated to 45°C to thereby dissolve the crude crystals in ethanol and then left to cool. After precipitation of crystals was confirmed, isopropyl ether (25 mL) was added to the mixture, and the resultant mixture was stirred for 2 hours. The mixture was cooled to 10°C or lower and then stirred for 1.5 hours. The crystals that precipitated were collected through filtration and washed with isopropyl ether. The crystals were dried under reduced pressure at 40°C, to thereby give trans-4-((4S)-methoxy-(2S)-pyrrolidinylmethoxy)cyclohexanecarboxylic acid ethyl ester·(-) camphorsulfonic acid salt (4.6 g, 91.0%) as white crystals.

### [Example 12]

### 2,5-Dichloro-4-[(1-methylindol-3-yl)carboxamido]phenylacetic acid

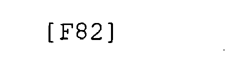

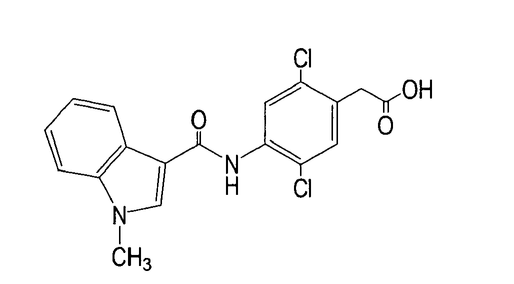

2,5-Dichloro-4-[(1-methylindol-3-yl)carboxamido]phenylacetic acid ethyl ester (20 g), methanol (100 mL), and 1mol/L sodium hydroxide (100 mL) were added to a flask, and the mixture was refluxed for 1.5 hours. Completion of reaction was confirmed through HPLC. Acetic acid (14.1 mL) was added to the reaction mixture at an internal temperature of 70°C, and the resultant mixture was stirred for a while until the temperature of the mixture was cooled to room temperature. The crystals that precipitated were collected through filtration, washed with water, and dried, to thereby give 2,5-dichloro-4-[(1-methylindol-3-yl)carboxamido]phenylacetic acid (16.75 g, 90.0%).

### [Example 13]

### trans-4-((2S,4S)-1-{2,5-dichloro-4-[(1-methylindol-3-yl)carboxamido]phenyl}acetyl-4-methoxypyrrolidin-2-yl)methoxycyclohexane-1-carboxylic acid ethyl ester

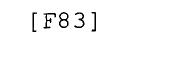

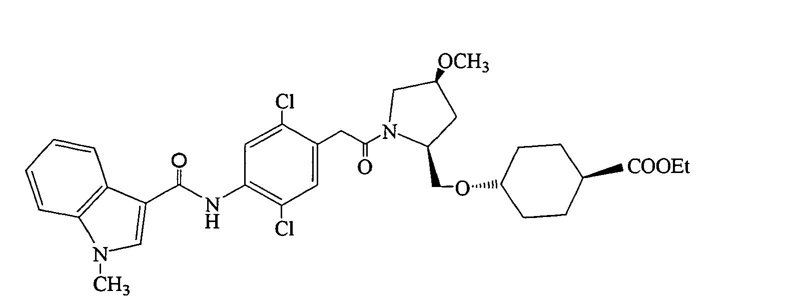

The compound obtained in Example 12 (25.00 g), the compound obtained in Example 9 (35.00 g), 1-hydroxybenzotriazole (10.75 g), and acetonitrile (250 mL) were added to a flask. Under stirring, triethylamine (20.3 mL) was added to the mixture at room temperature. After the mixture had been confirmed to become a solution, 1-ethyl-3-(3-dimethylaminopropyl)carbodiimide hydrochloride (17.79 g) was added to the mixture, and the resultant mixture was stirred for 5 hours at room temperature by means of a mechanical stirrer. Completion of reaction was confirmed through HPLC. Water (200 mL) was added to the reaction mixture. After stirring for a while, the crystals that precipitated were collected through filtration, sequentially washed with water (100mL), water:acetonitrile (1:1) (100 mL), and water:isopropyl alcohol (1:1) (100 mL), and dried under reduced pressure at 50°C, to thereby give trans-4-((2S,4S)-1-{2,5-dichloro-4-[(1-methylindol-3-yl)carboxamido]phenyl}acetyl-4-methoxypyrrolidin-2-yl)methoxycyclohexane-1-carboxylic acid ethyl ester (40.27 g, 94.3%) [isomer 0.88%].
¹H-NMR(CDCl₃)δ: 1.14-1.33(m,6H),1.36-1.55(m,2H),1.92-2.14 (m,4H), 2.15-2.43 (m,2H), 3.18-3.35 (m, including 2s, at δ 3.30, 3.33, total 8H), 3.44-3.58 (m,2H), 3.62-4.03 (series of m, including s at δ 3.86, total 8H), 4.09 (q,J=6.8Hz,2H), 4.25 (m,1H), 7.19-7.45 (series of m,total 4H), 7.78 (s,1H), 8.13 (m,1H), 8.22 (brd,J=3.2Hz,1H), 8.77 (d,J=7.2Hz , 1H) .

### [Example 14]

### 4-Amino-2,5-dichlorophenylacetic acid

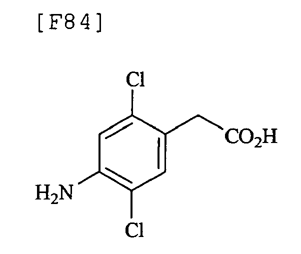

4-Amino-2,5-dichlorophenylacetic acid ethyl ester hydrochloride (10 g), methanol (30 mL), and 2N sodium hydroxide were added to a flask, and the mixture was stirred for 1 hour at 70°C. Completion of reaction was confirmed through HPLC, and the mixture was left to cool. Concentrated hydrochloric acid (10 mL) was added to the mixture, and the resultingt mixture was cooled with ice for 1 hour. The crystals that precipitated were collected through filtration, washed with water (30 mL), and dried under reduced pressure at 40°C, to thereby give 4-amino-2,5-dichlorophenylacetic acid (7.3 g, 95%).

### [Example 15]

### trans-4-[(2S,4S)-1-(4-Amino-2,5-dichlorophenyl)acetyl-4-methoxypyrrolidin-2-yl]methoxycyclohexane-1-carboxylic acid ethyl ester

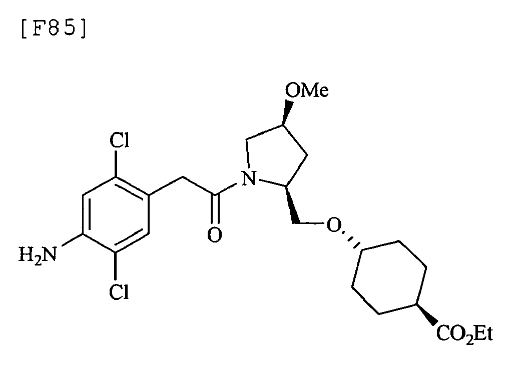

4-Amino-2,5-dichlorophenylacetic acid (6 g, 27.3 mmol) obtained in Example 14, trans-4-((4S)-methoxy-(2S)-pyrrolidinylmethoxy)cyclohexanecarboxylic acid ethyl ester·(-) camphorsulfonic acid salt (14.4 g), 1-hydroxybenzotriazole (4.43 g), and acetonitrile (120 mL) were mixed together.
Under stirring at room temperature, triethylamine (5.3 mL) was added to the mixture. After completion of dissolution, WSCI (7.33 g) was added to the mixture, and the resulting mixture was stirred. Completion of reaction was confirmed through HPLC. Water (80 mL) was added to the reaction mixture, and the resultant mixture was stirred. Seed crystals were added to the mixture to allow crystals to precipitate. Under cooling with ice, the mixture was stirred. The crystals that precipitated were collected through filtration, sequentially washed with water:acetonitrile (1:1) (10 mL) and water (20 mL), and dried under reduced pressure at 40°C, to thereby yield crystals (105 g, 79%).

The filtrate was concentrated under reduced pressure and then extracted with ethyl acetate. The extract was concentrated to dryness under reduced pressure, and acetonitrile (25 mL) was added to the residue, followed by stirring. Water (20 mL) was added to the mixture. Seed crystals were added to the mixture to allow crystals to precipitate. Under cooling with ice, the mixture was stirred. The crystals that precipitated were collected through filtration, sequentially washed with water:acetonitrile (1:1) (2 mL) and water (4 mL), and dried under reduced pressure at 40°C, to thereby yield 2'nd crop crystals (1.8 g, yield 14%, quality 96%).
¹H-NMR (400MHz, CDCl3) δ: 1.24 (dt,J=7.2,4Hz,3H), 1.45(t,J=12Hz,2H), 1.95-2.08 (m,6H), 2.23 (ddt,J=24.4,11.6,3.6Hz,2H), 3.31 (d,J=11.2Hz,3H) ,3.44-4.30 (m,9H), 4.07 (d,J=8.4Hz,2H), 4.10 (ddd,J=14.4,7.2,2Hz,2H), 6.7 7 (d,J=5.2Hz, 1H), 7.17 (d,J=4.8Hz, 1H)

### [Example 16]

### trans-4-((2S,4S)-1-{2,5-Dichloro-4-[(1-methylindol-3-yl)carboxamido]phenyl}acetyl-4-methoxypyrrolidin-2-yl)methoxycyclohexane-1-carboxylic acid ethyl ester

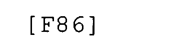

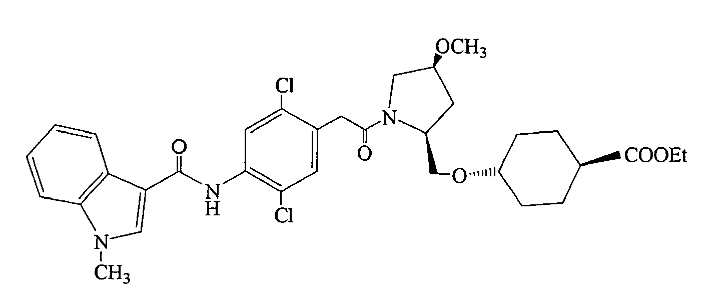

While 1-methylindole-3-carboxylic acid (150 mg, 0.86 mmol) and 1,2-dichloroethane (3 mL) were stirred under cooling on an ice-water bath, oxalyl chloride (0.095 mL, 1.07 mmol) was added to the mixture, and the resultant mixture was stirred for 1 hour at the same temperature. The reaction mixture was concentrated to dryness under reduced pressure. The thus-obtained crystals were dissolved in 1,2-dichloroethane (3 mL), and the solution was added to trans-4-[(2S,4S)-1-(4-amino-2,5-dichlorophenyl)acetyl-4-methoxypyrrolidin-2-yl]methoxycyclohexane-1-carboxylic acid ethyl ester (348 mg, 0.714 mmol) in 1,2-dichloroethane (15 mL) under stirring and under cooling. After completion of addition, the reaction mixture was refluxed for 10 hours under stirring. The reaction mixture was cooled, washed with water, and dried over anhydrous sodium sulfate. The solvent was removed under reduced pressure. The residue was purified through silica gel column chromatography, to thereby give trans-4-((2S,4S)-1-{2,5-dichloro-4-[(1-methylindol-3-yl)carboxamido]phenyl}acetyl-4-methoxypyrrolidin-2-yl)methoxycyclohexane-1-carboxylic acid ethyl ester (350 mg, 76%) as a crystalline powder (in a fraction obtained through use of chloroform:ethyl acetate (9:1 to 3:1, v/v)). Spectral data obtained from the thus-produced compound were identical with those of the compound produced through the method described above.

### [Example 17]

### trans-4-((2S,4S)-1-{2,5-Dichloro-4-[(1-methylindol-3-yl)carboxamido]phenyl}acetyl-4-methoxypyrrolidin-2-yl)methoxycyclohexane-1-carboxylic acid sodium salt pentahydrate

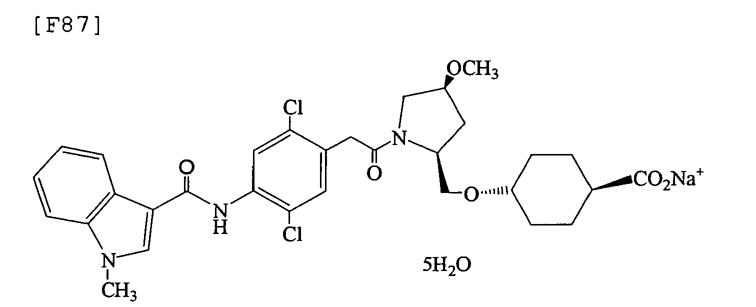

(1) Step for producing crude crystals
   To a 500-mL three-neck flask equipped with a mechanical stirrer, trans-4-((2S,4S)-1-{2,5-dichloro-4-[(1-methylindol-3-yl)carboxamido]phenyl}acetyl-4-methoxypyrrolidin-2-yl)methoxycyclohexane-1-carboxylic acid ethyl ester (20.00 g), isopropyl alcohol (200 mL), and 1N NaOH (34.1 mL) were added, and the mixture was refluxed for 2.5 hours (internal temperature 62 to 72°C). After completion of reaction was confirmed through HPLC, the reaction mixture was left to cool to 60°C. Isopropyl alcohol (100 mL) was added to the reaction mixture, and the resultant mixture was stirred for a while until the temperature of the mixture was lowered to room temperature. The crystals that precipitated were collected through filtration, washed with isopropyl alcohol, and air-dried, to thereby yield crude crystals (19.31 g).
(2) Recrystallization step
   The thus-obtained crude crystals (15.00 g) and 50% aqueous acetone (90 mL) were added to a flask, and the mixture was heated to 30 to 40°C, to thereby dissolve the crystals in acetone. Activated carbon (0.75 g) was added to the solution, and the mixture was stirred for 30 minutes.
   The mixture was subjected to filtration and washing with 10% aqueous acetone (10 mL). Acetone (360 mL) was added to the filtrate, and the mixture was gently stirred for 20 hours at room temperature by means of a mechanical stirrer. The crystals that precipitated were collected through filtration, washed with 10% aqueous acetone, and dried for 20 hours at 50°C (12.71 g). The crystals were hydrates-adjusted for 2 days, to thereby give trans-4-((2S,4S)-1-{2,5-dichloro-4-[(1-methylindol-3-yl)carboxamido]phenyl}acetyl-4-methoxypyrrolidin-2-yl)methoxycyclohexane-1-carboxylic acid sodium salt pentahydrate (14.18 g, 80.8%). Through X-ray powder diffraction, the compound was found to be of Type-II.
   ¹H-NMR (DMSO-d₆) δ: 1.09-1.43 (m,4H), 1.80-2.22 (m,7H), 3.10-4.30 (series of m, including s at δ 3.89, total 12H), 7.21 (dd,J=7.6,7.6Hz,1H), 7.28 (dd,J=7.6,7.6Hz,1H), 7.49 and 7 .52 (2S, total 1H), 7.56 (d,J=7.6Hz,1H), 7.89 and 7.90 (2S,total 1H),8.15(d,J=7.6Hz,1H),8.30(s,1H),9.37(s,1H)

## Claims

1. A method for producing a compound represented by formula (f): 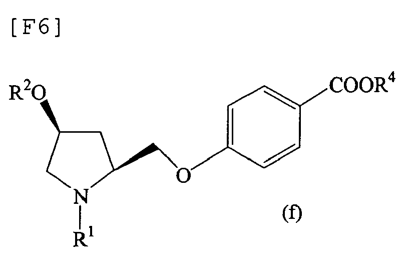 (wherein R¹ represents a protecting group for the amino group, R² represents a lower alkyl group, and R⁴ represents an alkyl group which may be substituted or an aralkyl group which may be substituted), **characterized by** comprising reacting an alkyl halide with a compound represented by formula (a): 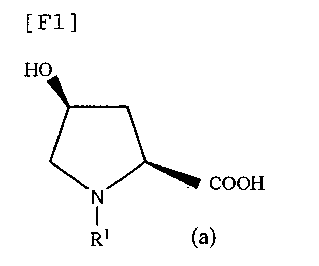 (wherein R¹ has the same meaning as defined above) in the presence of a base to thereby produce a compound represented by formula (b): 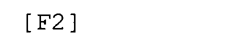 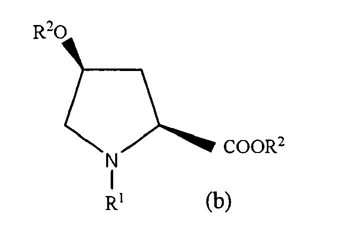 (wherein R¹ and R² have the same meanings as defined above); reacting a reducing agent with the compound represented by formula (b) to thereby produce a compound represented by formula (c): 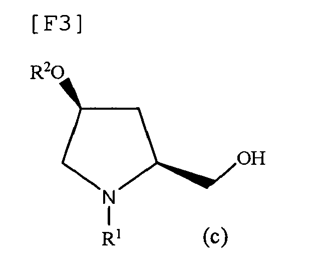 (wherein R¹ and R² have the same meanings as defined above); reacting the compound represented by formula (c) with an arylsulfonyl halide which may be substituted or an alkylsulfonyl halide which may be substituted in the presence of a base to thereby produce a compound represented by formula (d): 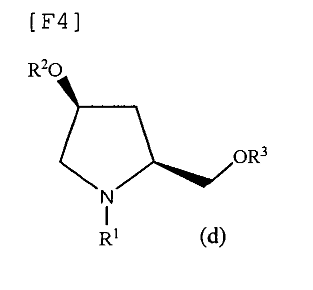 (wherein R¹ and R² have the same meanings as defined above, and R³ represents an arylsulfonyl group which may be substituted or an alkylsulfonyl group which may be substituted); and reacting the compound represented by formula (d) with a compound represented by formula (e): 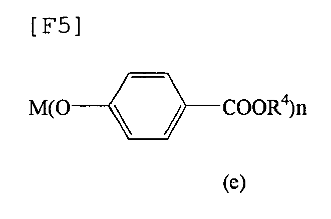 (wherein R⁴ has the same meaning as defined above, M represents an alkali metal atom or an alkaline earth metal atom, and n denotes an integer of 1 or 2).

2. A method according to claim 1, wherein R¹ represents a benzyloxycarbonyl group.

3. A method according to claim 1 or 2, wherein R² represents a methyl group or an ethyl group.

4. A method according to any one of claims 1 to 3, wherein R³ represents a para-toluenesulfonyl group or a methanesulfonyl group.

5. A method according to any one of claims 1 to 4, wherein the arylsulfonyl halide or the alkylsulfonyl halide is an arylsulfonyl chloride or an alkylsulfonyl chloride.

6. An oxalic acid salt of the compound represented by formula (g).

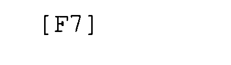

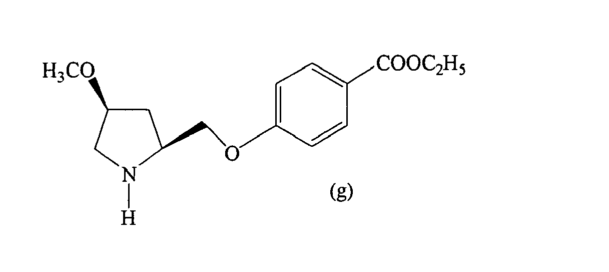

7. A method for producing a compound represented by formula (l): 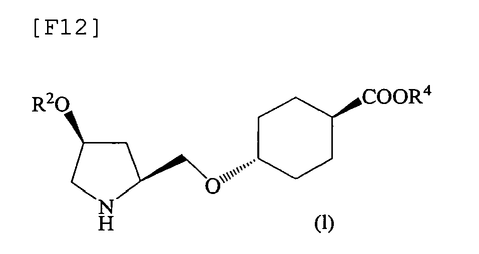 (wherein R² represents a lower alkyl group, and R⁴ represents an alkyl group which may be substituted or an aralkyl group which may be substituted), **characterized by** comprising reducing a compound represented by formula (h): 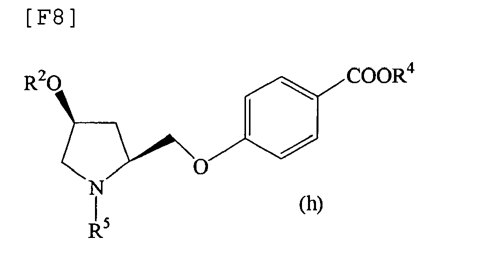 (wherein R² and R⁴ have the same meanings as defined above, and R⁵ represents a hydrogen atom or a protecting group for the amino group) to thereby produce a compound represented by formula (i): 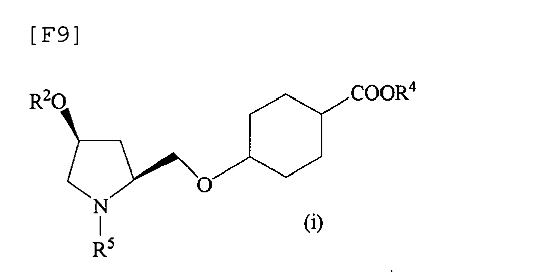 (wherein R², R⁴, and R⁵ have the same meanings as defined above); treating the compound represented by formula (i) with a base in an aprotic polar solvent and then reacting with water to thereby produce a compound represented by formula (j): 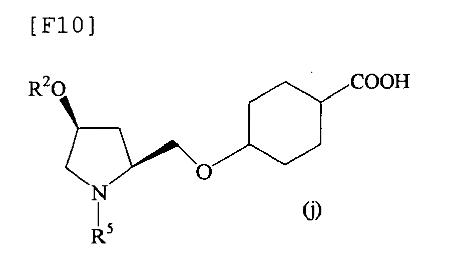 (wherein R² and R⁵ have the same meanings as defined above); and treating the compound represented by formula (j) with an acid in the presence of an alcohol to thereby produce a compound represented by formula (k): 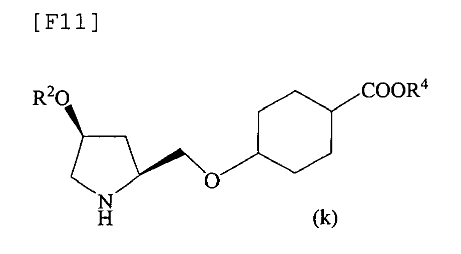 (wherein R² and R⁴ have the same meanings as defined above); and treating the compound represented by formula (k) with camphorsulfonic acid to thereby form an acid adduct salt so that an isomer of interest can be isolated.

8. A method for producing a compound represented by formula (l): 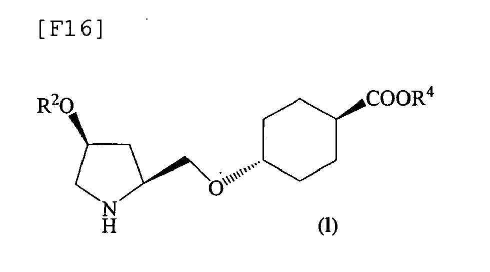 (wherein R² represents a lower alkyl group, and R⁴ represents an alkyl group which may be substituted or an aralkyl group which may be substituted), **characterized by** comprising reducing a compound represented by formula (h): 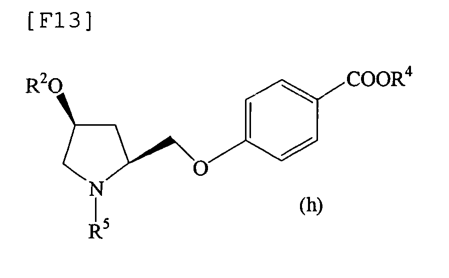 (wherein R² and R⁴ have the same meanings as defined above, and R⁵ represents a hydrogen atom or a protecting group for the amino group) to thereby produce a compound represented by formula (i): 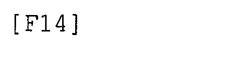 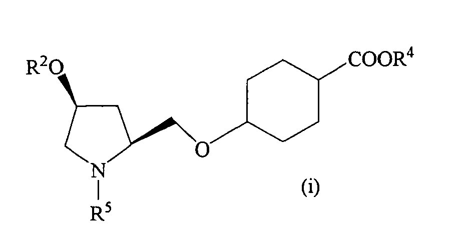 (wherein R², R⁴, and R⁵ have the same meanings as defined above); treating the compound represented by formula (i) with a base in an aprotic polar solvent and then reacting with water to thereby produce a compound represented by formula (j): 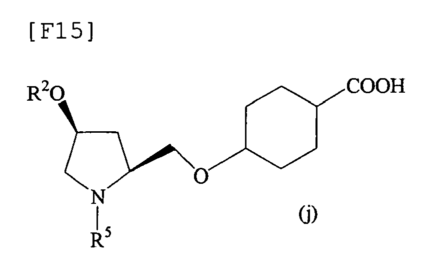 (wherein R² and R⁵ have the same meanings as defined above); and treating the compound represented by formula (j) with camphorsulfonic acid to thereby form an acid adduct salt so that an isomer of interest can be isolated.

9. A method for producing a compound represented by formula (l): 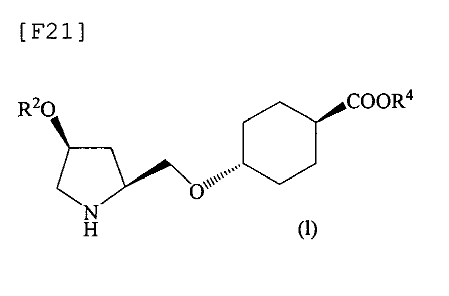 (wherein R² represents a lower alkyl group, and R⁴ represents an alkyl group which may be substituted or an aralkyl group which may be substituted), **characterized by** comprising reducing a compound represented by formula (h): 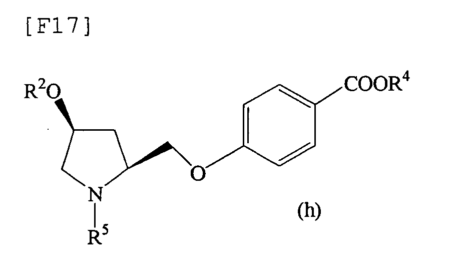 (wherein R² and R⁴ have the same meanings as defined above, and R⁵ represents a hydrogen atom or a protecting group for the amino group) to thereby produce a compound represented by formula (i): 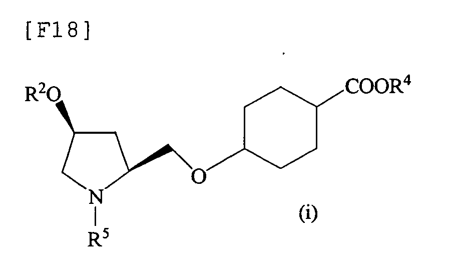 (wherein R², R⁴, and R⁵ have the same meanings as defined above); treating the compound represented by formula (i) with a base in an aprotic polar solvent and then reacting with water to thereby produce a compound represented by formula (j): 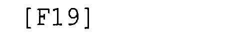 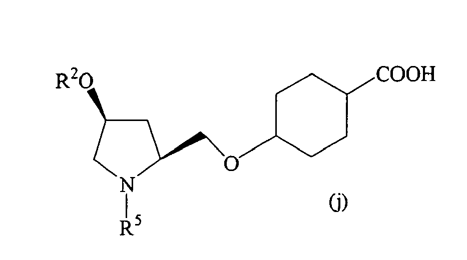 (wherein R² and R⁵ have the same meanings as defined above); isolating the compound represented by formula (j) as a salt and then treating the salt with an acid in the presence of an alcohol to thereby produce a compound represented by formula (k) : 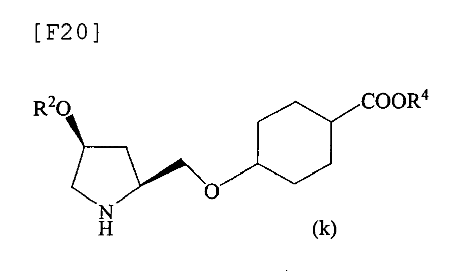 (wherein R² and R⁴ have the same meanings as defined above); and treating the compound represented by formula (k) with camphorsulfonic acid to thereby form an acid adduct salt so that an isomer of interest can be isolated.

10. A method for producing a compound represented by formula (l): 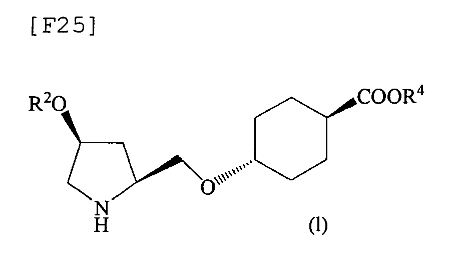 (wherein R² represents a lower alkyl group, and R⁴ represents an alkyl group which may be substituted or an aralkyl group which may be substituted), **characterized by** comprising reducing a compound represented by formula (h): 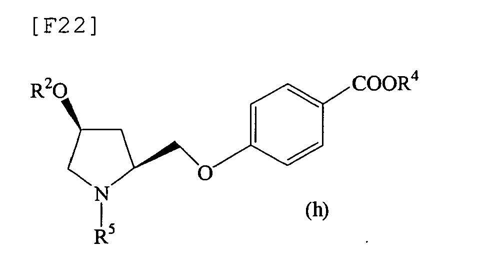 (wherein R² and R⁴ have the same meanings as defined above, and R⁵ represents a hydrogen atom or a protecting group for the amino group) to thereby produce a compound represented by formula (i): 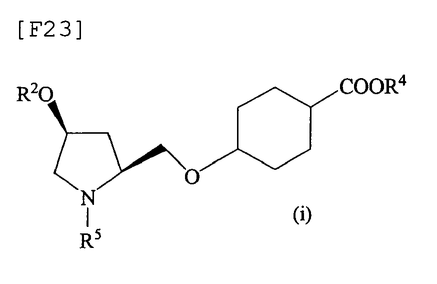 (wherein R², R⁴, and R⁵ have the same meanings as defined above); treating the compound represented by formula (i) with a base in an aprotic polar solvent and then reacting with water to thereby produce a compound represented by formula (j) : 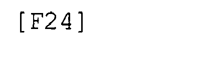 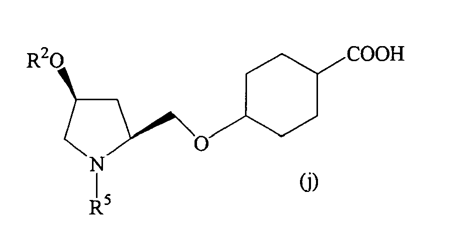 (wherein R² and R⁵ have the same meanings as defined above); isolating the compound represented by formula (j) as a salt and then treating the compound with camphorsulfonic acid to thereby produce an acid adduct salt so that an isomer of interest can be isolated.

11. A method according to any one of claims 7 to 10, wherein the compound represented by formula (h) is a compound produced through a method according to claim 1, a compound produced through removal of the protecting group for the amino group of a compound produced through a method according to claim 1, or a compound produced through removal of the protecting group for the amino group of a compound produced through a method according to claim 1 and then protection of the amino group with a protecting group which differs from the removed protecting group.

12. A method according to any one of claims 7 to 11, wherein R⁵ represents a tert-butoxycarbonyl group.

13. A method according to any one of claims 7 to 12, wherein R⁴ represents a methyl group or an ethyl group.

14. A method according to any one of claims 7 to 13, wherein the base is sodium hydride, lithium hydride, or potassium t-butoxide.

15. A method according to any one of claims 7 to 14, wherein the aprotic polar solvent is N,N-dimethylformamide or N,N-dimethylacetamide.

16. A camphorsulfonic acid salt of the compound represented by formula (m).

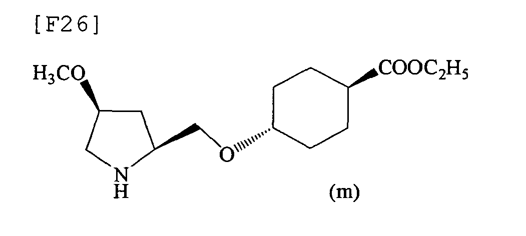

17. A method for producing a compound represented by formula (o): 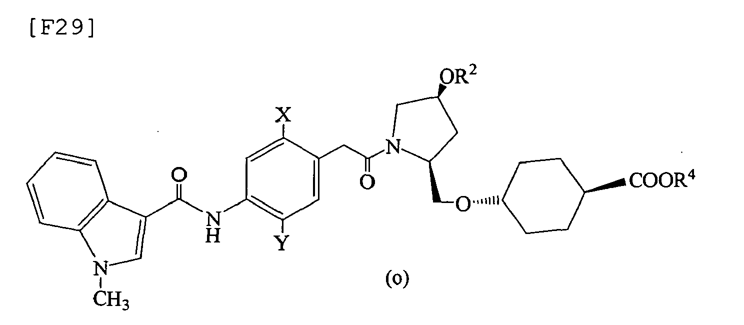 (wherein R² represents a lower alkyl group, R⁴ represents an alkyl group which may be substituted or an aralkyl group which may be substituted, X represents a hydrogen atom or a halogen atom, and Y represents a halogen atom or a lower alkoxy group), **characterized by** comprising reacting a compound represented by formula (l) : 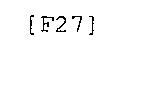 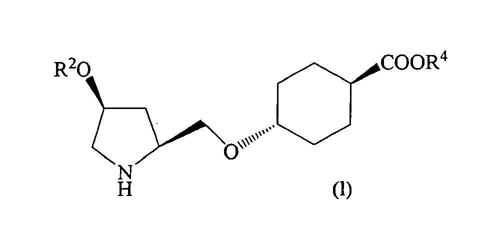 (wherein R² and R⁴ have the same meanings as defined above) produced through a method according to any one of claims 7 to 10, with a compound represented by formula (n): 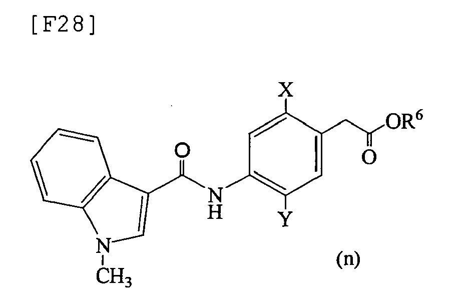 (wherein R⁶ represents a hydrogen atom, a linear or branched lower alkyl group which may be substituted, or an aralkyl group which may be substituted, and X and Y have the same meanings as defined above).

18. A method for producing a compound represented by formula (p): 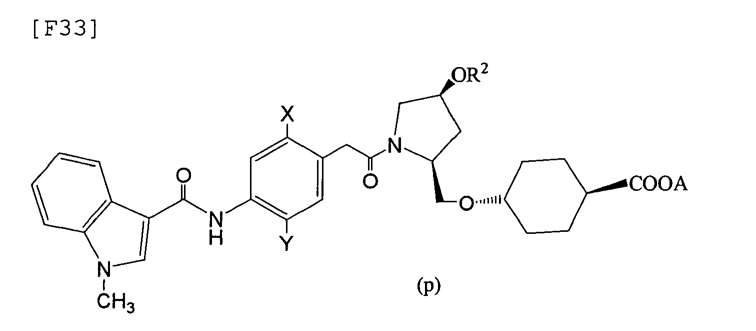 (wherein R² represents a lower alkyl group, A represents a hydrogen atom, an alkali metal atom, an alkaline earth metal atom, or an organic amine, X represents a hydrogen atom or a halogen atom, and Y represents a halogen atom or a lower alkoxy group) or a hydrate thereof, **characterized by** comprising reacting a compound represented by formula (l): 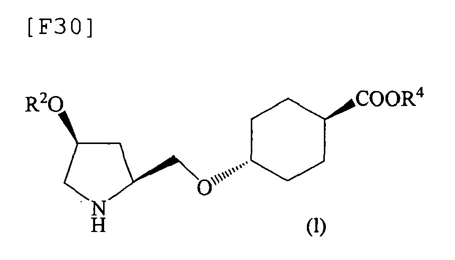 (wherein R² has the same meaning as defined above, and R⁴ represents an alkyl group which may be substituted or an aralkyl group which may be substituted) produced through a method according to any one of claims 7 to 10, with a compound represented by formula (n): 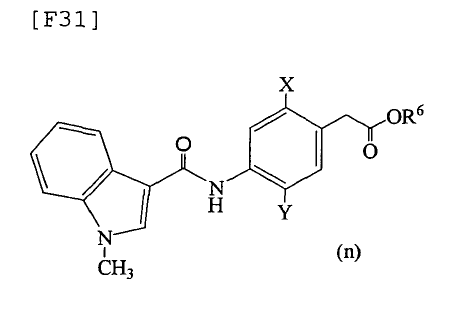 (wherein R⁶ represents a hydrogen atom, a linear or branched lower alkyl group which may be substituted, or an aralkyl group which may be substituted, and X and Y have the same meanings as defined above) to thereby produce a compound represented by formula (o): 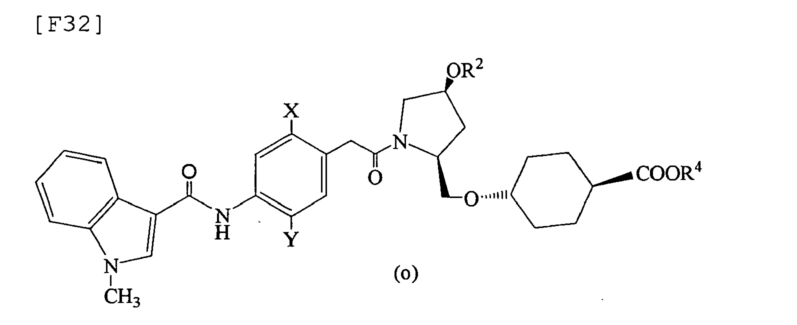 (wherein R², R⁴, X, and Y have the same meanings as defined above); and hydrolyzing the compound represented by formula (o).

19. A method for producing a compound represented by formula (o): 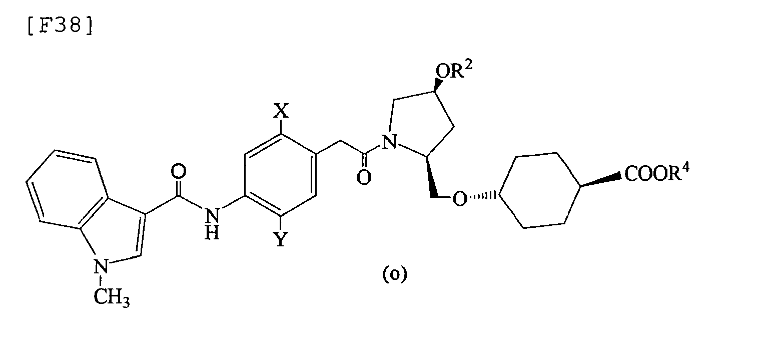 (wherein R² represents a lower alkyl group, R⁴ represents an alkyl group which may be substituted or an aralkyl group which may be substituted, X represents a hydrogen atom or a halogen atom, and Y represents a halogen atom or a lower alkoxy group), **characterized by** comprising reacting a compound represented by formula (l): 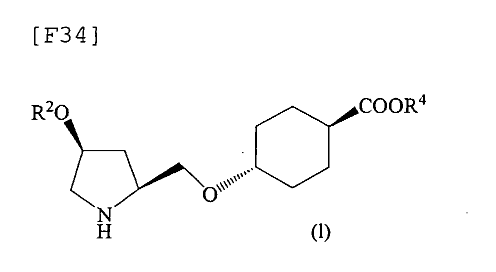 (wherein R² and R⁴ have the same meanings as defined above) produced through a method according to any one of claims 7 to 10, with a compound represented by formula (s): 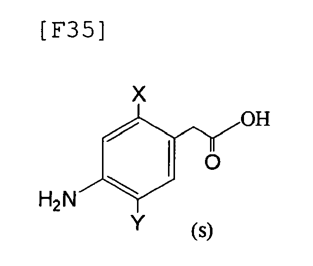 (wherein X and Y have the same meanings as defined above) to thereby produce a compound represented by formula (t): 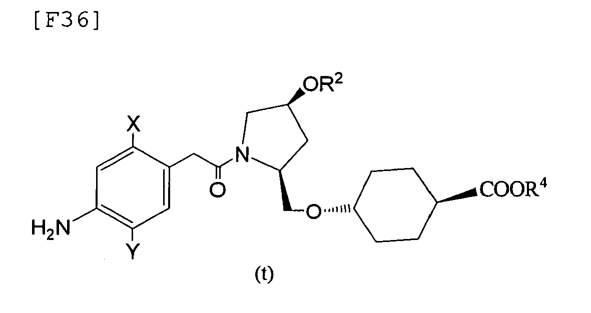 (wherein R², R⁴, X, and Y have the same meanings as defined above); and reacting the compound represented by formula (t) with a compound represented by formula (u).

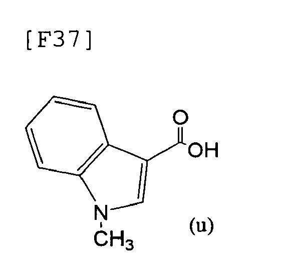

20. A method for producing a compound represented by formula (p): 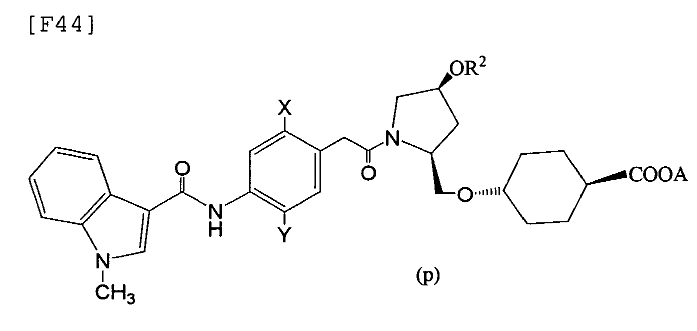 (wherein R² represents a lower alkyl group, A represents a hydrogen atom, an alkali metal atom, an alkaline earth metal atom, or an organic amine, X represents a hydrogen atom or a halogen atom, and Y represents a halogen atom or a lower alkoxy group) or a hydrate thereof, **characterized by** comprising reacting a compound represented by formula (l): 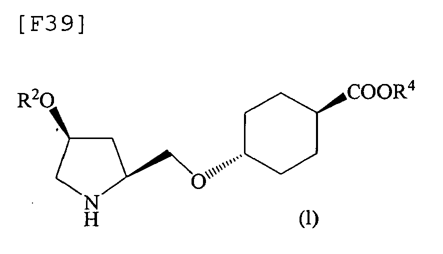 (wherein R² has the same meaning as defined above, and R⁴ represents an alkyl group which may be substituted or an aralkyl group which may be substituted) produced through a method according to any one of claims 7 to 10, with a compound represented by formula (s): 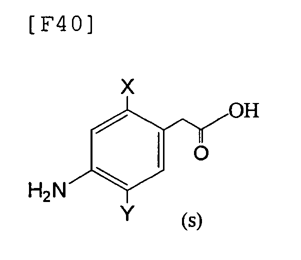 (wherein X and Y have the same meanings as defined above) to thereby produce a compound represented by formula (t): 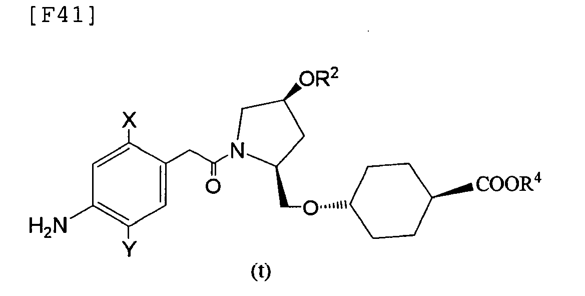 (wherein R², R⁴, X, and Y have the same meanings as defined above); reacting the compound represented by formula (t) with a compound represented by formula (u): 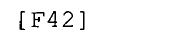 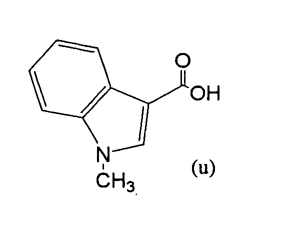 to thereby produce a compound represented by formula (o): 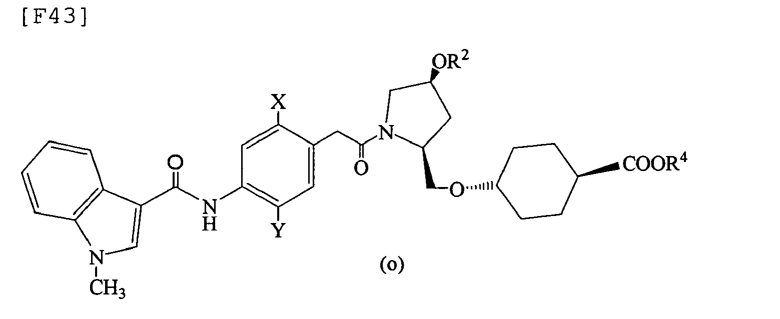 (wherein R², R⁴, X, and Y have the same meanings as defined above); and hydrolyzing the compound represented by formula (o).

21. A method according to any one of claims 17 to 20, wherein R² represents a methyl group or an ethyl group.

22. A method according to any one of claims 17 to 21, wherein X represents a chlorine atom or a fluorine atom.

23. A method according to any one of claims 17 to 22, wherein each of X and Y represents a chlorine atom.

24. A method according to any one of claim 18 and claims 20 to 23, wherein A represents sodium.
